# EUROPEAN PATENT APPLICATION

(11) **EP 4 002 375 A1**
(43) Date of publication of application: **25.05.2022**
(21) Application number: 20208936.3
(22) Date of filing: 20.11.2020
(51) Int. Cl.: G16B 40/10

(54) **MASS SPECTROMETRY SYSTEM AND METHOD FOR PROVIDING AN INDICATION OF INTEGRITY OF A BIOLOGICAL SAMPLE**

(71) Applicant: OmicEra Diagnostics GmbH, 82152 Planegg (DE)
(72) Inventor: GEYER, Philipp Emanuel, 82152 Planegg (DE); WINTER, Sebastian Virreira, 82152 Planegg (DE); STRAUSS, Maximilian Thomas, 82152 Planegg (DE); DOLL, Sophia, 82152 Planegg (DE)
(74) Representative: Sackin, Robert

(57) **Abstract**

A mass spectrometry system comprises a mass spectrometry apparatus configured to provide mass spectrometry data of a biological sample; and a computer system configured to process the mass spectrometry data to determine a characteristic of the biological sample as integrity data of the biological sample; and output an indication of the similarity to a predetermined sample characteristic as integrity data of the biological sample. A computerized method provides an indication of integrity of a biological sample from mass spectrometry data, the computerized method comprising: processing mass spectrometry data of a biological sample to determine a sample characteristic of the biological sample; and outputting an indication of the similarity to a predetermined sample characteristic as integrity data of the biological sample.

## Description

### Field of Invention

The present invention relates to a mass spectrometry system and method for providing an indication of integrity of a biological sample from mass spectrometry data.

### Background

In a laboratory or clinical environment, it is of utmost importance that the integrity of a sample that is analysed to retrieve information about this sample or about an individual is not lost or corrupted. In other words, it is critical that the integrity of a sample can be reliably assessed. Integrity is understood to relate to the quality of being whole and complete, *i.e*. internal consistency, such that there is no corruption of information. Sample integrity can also be seen as a proxy for the quality of a sample. The integrity of a sample can be corrupted due to various reasons including, but not limited to:
(A) unknown or corrupted origin of a sample
(B) losing the identity of a sample
(C) potential contamination of a sample
(D) problems in sample processing
(E) degradation of a sample

If the integrity cannot be confirmed, the generated data might be corrupt or cannot be assigned to the biological system and no usable knowledge can be generated. The present invention applies a mass spectrometry system to confirm the integrity of a biological sample.

There are no known methods which are able to verify multiple aspects of sample integrity as described above at the same time. Known methods for verifying e.g. the integrity of a sample include a physical barcode applied to a receptacle containing the sample. For example, a doctor applies a physically barcode with a printer to a blood collection container. The barcode contains information about the origin of a sample and in turn allows to reassign the sample to the individual of which it was obtained. The blood in the container may be the sample. The barcode can be read with a reader and can be assigned to this individual. This allows to match the sample and any generated data from this sample to the individual. Such physical barcodes can be used to identify the sample and its origin. However, known barcodes cannot be used to check the integrity of the sample. A sample specific identifier which cannot be lost can only be generated from the sample itself.

If the barcode label is lost or the sample is degraded and/or contaminated, data that are generated from the sample and the sample itself will be rendered useless or result in the wrong conclusions. These are examples for the corruption of the integrity of a sample. Other known methods for verifying parts of the integrity of a sample include DNA or RNA sequencing. DNA or RNA sequencing cannot quantify proteins, let alone peptides, post translational modification, metabolites, lipids and ions of ions. The genome and transcriptome is particularly difficult to analyse in samples that do not contain any cells or a very low number of cells such as body fluids like urine, cerebrospinal fluid, plasma and serum. DNA- or RNA-based analysis may help to identify the origin of a sample but it does not help to quantitatively determine contaminations with other samples or assess its integrity. Moreover, sequencing technologies are not able to detect quality issues introduced by sample collection or pre-processing of the samples.

For these reasons, there is a need for a reliable and accurate system and a method for providing verification of the integrity of a sample.

### Background mass spectrometry

Mass spectrometry systems are highly complex instrumentations. They comprise a large number of components that focus an ion beam with lenses usually in vacuum, manipulate the ions flight path by dynamic electric fields, allow to filter for ions with certain mass to charge (m/z) ratios and in some mass spectrometers also ion mobility or collisional cross section and break them into smaller fragments at selectable energies. The determination of an ion's m/z, ion mobility or collisional cross section, its intensity signal, its charge and its fragmentation pattern can be used for identification and quantification. Many types of mass analysers and detectors are used for this purpose. A typical MS-measurement uses two steps (MS1 and MS2) to acquire the necessary information for identification of the biomolecules. In the MS1 step (full scan), a broad-range mass spectrum (e.g. m/z=300-1650 Th) is acquired, delivering m/z values for all intact biomolecule masses at a distinct time point during the LC run. In the MS2 scan, a single ion species in data dependent acquisition (DDA) or a broader mass range in data independent acquisition (DIA) or other scan types is selected and fragmented and the fragments masses are determined. The MS1 and MS2 measurements deliver the data used for the identification and quantification of biomolecules. However, also an MS1 or a MS2 spectrum alone can be used to identify a biomolecule. If the biomolecule of interest is a peptide, its sequence can be determined as peptides are combinations of amino acids with distinct masses. The MS1 spectrum provides the intact peptide mass and thus constrains the peptide's amino acid composition. Typically, all potentially generated peptides are calculated in-silico from a database that is built from the genome sequence of the organism analysed using the known enzyme specificity. For all peptide sequences in the database with a compatible mass that satisfies the enzyme specificity, theoretical MS2 spectra are determined by in-silico digestion and measured spectra are compared to these theoretical spectra to identify the best hit based on the number of fragment ions that match. However, the sequences can also be determined without comparing the measured spectra to theoretical spectra from a database, which is called de novo sequencing. In the database search strategy, the number of matches to the measured fragments is converted to a score that reflects the likelihood that these matches occurred by chance. False Discovery Rates (FDRs) are then rigorously determined by comparison to the number of peptide and protein matches in a sequence reversed database. Additionally, the sequence of entire proteins can be determined without prior cleavage into peptides. Usually peptides are easier to ionize and therefore easier to be transferred into gas phase which allows the determination of their m/z ratio. The MS data acquisition can be targeted by acquiring fragment mass spectra of biomolecules with previously determined characteristics (e.g. m/z, ion mobility, retention time, charge) or untargeted by acquiring a broad range of fragment mass spectra.

In living organisms, the genetic sequence information provides a blueprint for the synthesis of proteins and peptides. Between individuals of the same species, there are usually multiple variants of individual gene sequences that vary in their sequence due to mutations in the genetic code. A substitution of a single nucleotide at a specific sequence in the genome is called single nucleotide polymorphism (SNP). Among the variants, non-synonymous ones can result in amino acid changes that are called single amino acid polymorphisms (SAPs). Genetic variants can further be caused by insertions, deletions, tandem repeats, inversions, translocations, duplications, missense mutations and others. Genetic variants can further be generated by all kinds of mutations such as germline or somatic mutations.

Genetic variants of proteins can be investigated directly on the proteome level by the technology of mass spectrometry (MS)-based proteomics that allows the analysis of peptide and protein sequences. A mass spectrometer can determine the mass to charge ratio (m/z) of whole proteins or peptides. Applying a database search, the mass spectra can be interpreted to determine the amino acid sequence of the proteins or peptides or both. As the amino acid sequence can be determined, the technology MS-based proteomics is also able to identify all kinds of amino acid variations that are determined by different alleles but also by splice forms and other post translational modifications of proteins. For example, two humans may have a different life history, different lifestyle and also a different genetic background. These and other factors may influence how the two individuals will look like and how they differ phenotypically by eye, but also on a molecular level. The composition of biological molecules might be different. For example, the one individual may live a healthy and the other one a rather unhealthy lifestyle, *that can result* in different blood cholesterol levels.

Another way how individuals may vary from each other is by their genetic code. Individuals differ from each other by the composition of their genome, including gene sequences and non-coding regions. Deoxyribonucleic acid (DNA) and ribonucleic acid (RNA) are the prior molecules that are analysed to retrieve information about an organism's genome sequences. This is usually done by sequencing technologies that allow to uncover an extensive amount of genetic variation in humans, including various single nucleotide variations, insertions, deletions, tandem repeats, inversions, translocations and duplications. The category of these technologies are called genomics and transcriptomics, respectively, if DNA or RNA is analysed. In each generation of a species, mutations accumulate over time and through recombination, independent assortments and zygote formation, each individual within a species will have a unique combination of alleles. The closer the relationship of two species or two individuals within the same species, the more similar is their genome or genetic relationship. Two closely related individuals will have a very similar genome. As the DNA is transcribed into RNA, RNA is subsequently translated into proteins. As each individual has its own set of alleles which vary among others in single nucleotide polymorphisms (SNPs) that occur roughly every 1000 base pairs. The relationship or comparison of genomes is widely applied. Application fields of genomics and transcriptomics are population genetics, analysis of mutations in cancer, diagnosis by analysing disease associated or disease relevant genes, paternity testing, forensic and many more. As each individual has its own unique genome, next to analysing single genes or the comparison of genomes, one can also use the genomes to identity individuals, which is applied across various fields, but especially in forensic. Thus far, millions of human SNPs or mutations have been identified. These variants may strongly correlate with phenotypic variations of diseases and other traits. Proteins consist of 20 different amino acids and the sequence of these amino acids is encoded in the genome. If a gene is mutated with one of the various possible alterations, the sequence of the protein may also change. SNPs can result in changes of amino acids, changes of parts or even the entire protein sequence compared to the other alleles and their encoded protein. Usually, genomic and transcriptomic methods are applied to identify SNPs.

Next to DNA, the concentrations of other biomolecules such as the above-mentioned cholesterol levels or the levels of proteins, lipids, metabolites and peptides can be different between individuals. While the DNA is encoded from the beginning for each organism and even each individual (apart from acquired mutations), the presence, absence and concentrations of the other biomolecules can change in different conditions. For example, the genetic background of a person, its life style and its history can affect these biomolecules but they might be specific for an individual even on the long-range. Distinct biomolecules can present different levels of these biomolecules in plasma that can be seen as "individual-specific" as they keep their levels relatively constant even over years.

### Terminology

Mass spectrometer = all kinds of mass spectrometer
MS = mass spectrometer, mass spectrometric, mass spectrometry
Mass spectrometry data = data generated by a mass spectrometry system. This unprocessed and further processed data. Such data may be one or more of mass, charge, mass-to-charge-ratio, fragment spectra, MS2 fragment information, ion mobility, intensity information, retention time, sequence (in case of proteins/peptides) or other identity (in case of lipids/metabolites), information on peptide, protein, post translational modification, allele, biomolecules such as lipids, small molecules such as drugs or metabolites, carbohydrates, preferably quantified biomolecules.
Predetermined sample characteristic = is a characteristic of a sample or its biological origin. The predetermined sample characteristic can be defined or determined before or after the mass spectrometer analysis that is used to confirm the sample integrity.
LC = liquid chromatography, all kinds of LCs, also high-performance liquid chromatography or ultra high-performance liquid chromatography (HPLC/UHPLC); Other peptide and/or protein separation methods include for example gas phase separation/fractionation and capillary electrophoresis
   The term "tissue" refers to all biological tissues, including a sample of human origin, human remains or mummies, or non-human sources, such as zoological and/or preserved remains. It includes liquid and solid tissues as well as tissues fixed with different fixation methods.
   A biopsy is a sample that is taken from an organism. The biopsy can originate from a tissue sample or a bodily fluid. In the latter case the biopsy is often referred to as a liquid biopsy, but also in general a biopsy.
Biological system = A biological sample may be anything of biological origin. For example, a human individual, animal, environment such as piece of soil, a part of a larger biological system such as an organ of a human individual. The presence, absence, composition or concentrations of biomolecules may be unique for a distinct biological system and may be determined from a sample of this biological system.
Sample = a limited quantity of something which is intended to be similar to and represent a larger amount of a biological system. A sample can be a biopsy, tissue sample, liquid biopsy such as blood, plasma, urine, cerebrospinal fluid or processed part or previous part of a biological system such as plasma which is obtained from processing blood by centrifugation or FFPE tissue which is a processed tissue. Such a biological sample will always contain distinct molecules for example DNA, RNA, proteins, metabolites and/or lipids.
Biomolecule = any kind of molecule present in a living organism such as proteins, peptides, lipids, metabolites, carbohydrates, modifications of these Digestion and cleavage are used vice-versa
Blood, plasma, serum and all their related matrices are used vice-versa
Feature: Ion measured in the MS, independent of its identification (however: preferably associated with fragment information)
Metadata = all available data; For example, clinical chemistry analysis, anthropometrics, information on ethical origin, genomic data, transcriptomic data, proteomic data
PTM = Post translational modifications such as phosphorylation, glycosylation, glycation, ubiquitination, S-nitrosylation, methylation, N-acetylation, SUMOylation, and/or lipidation
m/z = mass to charge ratio
MS-integrity-code = mass spectrometry-integrity-code. One or more predetermined sample characteristics that can be analysed by a mass spectrometry system. The MS-integrity-code may include mass spectrometer data and/or other predetermined sample characteristics.
AI = Artificial intelligence, machine learning, deep learning

### Summary of Invention

According to a first aspect of the invention, there is provided a mass spectrometry system, the mass spectrometry system comprising: a mass spectrometry apparatus configured to provide mass spectrometry data of a biological sample; and a computer system configured to: process the mass spectrometry data of a biological sample to determine a determined sample characteristic of the biological sample as integrity data of the biological sample; and output the integrity data of the biological sample.

Generally, the expression "determine" in this context is understood to mean the acquisition of qualitative and/or quantitative data. The expression "predetermined sample characteristic" is a characteristic of a sample or its biological origin. The predetermined sample characteristic can be defined or determined before or after the mass spectrometry analysis that is used to confirm the sample integrity. The predetermined sample characteristic is the information to which data of the mass spectrometry system is compared to. The integrity data may comprise a determined sample characteristic. The determined sample characteristic may be measured by a mass spectrometry apparatus. The integrity data may comprise an indication of the similarity of the determined sample characteristic and the predetermined sample characteristic.

Similarity may be generally defined as a similarity measure that quantifies the similarity between two datasets, meaning that they may take on large values for similar datasets and small or negative values for dissimilar datasets. Similarity can either be defined on the actual similarity (e.g., Pearson's correlation or Cosine similarity) or from the inverse of a distance metric (e.g., Manhattan or Euclidean distance). In this context, we define datasets to be overlapping when they exceed a fixed similarity threshold. Furthermore, we can combine multiple similar measurements to a single reference where individual datasets are compared against. The dataset that are compared can contain a predetermined sample characteristic and/or a mass spectrometry determined sample characteristic. Here, a predetermined sample characteristic can be used to infer the expected mass spectrometry data and increase the sensitivity of a similarity check. Similarity, according to aboves definition may also be calculated between categorical and numerical data, e.g. when transforming categorical to numerical data and scaling before a similarity is calculated. Here, a reference value or a reference measurement might be used to transform or scale the data. Accordingly, one can assign a similarity metric for e.g. an overlap of alleles when transforming them into a vector and calculating the distance. For example the sex woman can be expressed as a similarity if a high level of the pregnancy zone protein (PZP) is detected.

The integrity data may comprise an indication of the relationship between the determined sample characteristic and the pre-determined sample characteristic.

Advantageously, mass spectrometry can read out an extensive array of biomolecules, providing an extensive amount of data that describe the sample of which the data are generated from. A mass spectrometry system can provide data even with single measurements and can provide even higher coverage of biomolecules by multiple measurements to provide data of a biological sample. The generated data of biomolecules can be applied to assess the integrity of a biological sample by comparing the mass spectrometry generated data with one or more predetermined sample characteristics. This data can be seen as a code reflecting the integrity of a sample and this code can be read by a mass spectrometry system. Hence, the code is called a mass spectrometry-integrity-code or MS-integrity-code A mass spectrometry system can further provide data that can evaluate a predefined MS-integrity-code from non-mass spectrometry generated data. This is possible due to the extensive amount of information about biomolecules that can be generated with a MS analysis. This non-MS data include but are not limited to data on sex, age, immunoassay measurements, other laboratory medicine analysis or the genome of an individual. Advantageously, the integrity of a biological sample can be assessed by mass spectrometry with regards to the origin of the sample for example to track a sample, to match information to a sample, to match one or multiple sample to a distinct individual, to match of one or multiple samples to each other, to match of one or multiple samples to distinct clinical parameter, to identify an individual, to determine the similarity of two or more samples, to confirm or exclude a close similarity of two or more samples, to determine if two or more samples are from the same type of sample (e.g. plasma vs. urine; liver vs. muscle), to detect alterations of the sample integrity by contamination with another sample, to detect alterations of the sample integrity by problems with the sample quality e.g. problems due to sample processing or storage, to detect alterations of the sample integrity by problems of the mass spectrometry system. Advantageously, the analysis of the sample integrity of one or multiple samples can also be applied to determine the layout and/or arrangement of samples in a physical metrics such as a 96 or 384 array and/or labware.

The mass spectrometry system may also comprise a sample preparation method for mass spectrometry analysis, a pre-separation method not coupled to a mass spectrometer, a pre-separation method coupled to a mass spectrometer. The MS-integrity-code may also contain information on the performance of these technical components such as a successful sample preparation, the proper function of the separation method or proper ionization of the biomolecules.

The computer system may comprise one or more of the following units: a unit for generating a MS-integrity-code based on a predetermined sample characteristic; a unit for generating data from the mass spectrometer analysis that reflect a sample characteristic of the MS-integrity-code; a unit for generating a similarity metrics between a predetermined sample characteristic in the MS-integrity-code and mass spectrometry data; a unit for comparing a MS-integrity-code and mass spectrometry data to identify a sample; a unit for comparing a MS-integrity-code and mass spectrometry data for quality assessment of a sample; a unit for comparing a MS-integrity-code and mass spectrometry data to detect sample contamination; a unit for comparing a MS-integrity-code and mass spectrometry data for controlling sample taking and processing; a unit for identification of the sample; a unit for quality assessment of a sample; a unit for quality assessment of sample processing. The computer system is configured to process the mass spectrometry data to identify directly and/or indirectly a predetermined characteristic of the biological sample as integrity data of the biological sample. This is because some characteristics can directly be measured such as protein levels, whereas other characteristics such as sex can only be indirectly measured by proteins indicating the sex. Other factors such as HDL levels might correlate with proteins in plasma.

Optionally, next to bottom-up proteomics, top-down and native proteomics can be applied to check sample integrity. Top-down/native proteomics has the advantage that no digestion of a protein into peptides has to be performed before the mass spectrometry analysis.

According to a second aspect of the invention, there is provided a method, preferably a computerized method, for providing an indication of integrity of a biological sample from mass spectrometry data, the computerized method comprising: processing mass spectrometry data of a biological sample to determine a determined sample characteristic of the biological sample as integrity data of the biological sample; and outputting the integrity data of the biological sample.

Optionally, the integrity data comprises the determined sample characteristic and/or an indication of similarity of the determined sample characteristic of the biological sample and a predetermined sample characteristic.. The integrity data may comprise the determined sample characteristic. Additionally or alternatively, the integrity data may comprise an indication of similarity of the determined sample characteristic of the biological sample and a predetermined sample characteristic.

Optionally, the biological sample is a human biological sample, and/or the biological sample comprises one or more of blood, plasma, serum, urine, cerebrospinal fluid, saliva, tears, stool, gastric juice, tissue, fresh tissue, fixed tissue, e.g. formalin-fixed paraffin-embedded tissue, processed tissue, biopsies, liquid biopsies, hair, and/or bone. The biological sample may be a human biological sample. The biological sample may comprise a human biological sample. Additionally or alternatively, the biological sample comprises one or more of blood, plasma, serum, urine, cerebrospinal fluid, saliva, tears, stool, gastric juice, tissue, fresh tissue, fixed tissue, e.g. formalin-fixed paraffin-embedded tissue, processed tissue, biopsies, liquid biopsies, hair, and/or bone. Preferably, the biological sample comprises one or more of blood, plasma, serum, urine, cerebrospinal fluid (CSF), saliva, tears, stool, gastric juice, a processed liquid sample such as plasma or serum depleted from the highest abundant proteins or a plasma after enrichment of subpopulation such as low abundant proteins, tissue (fresh, frozen, embedded such as FFPE), processed tissue, biopsies, liquid biopsies, hair, and/or bone. Advantageously, the biological sample contains an extensive amount of biomolecules such as proteins, peptides, lipids, metabolites, drugs, DNA and RNA that can be read out by a mass spectrometry system. Optionally, one or more samples may be used. Optionally, these biomolecules may correlate and/or these biomolecules may be connected to other sample characteristics such as anthropometrics data, disease status or sex.

Optionally, the predetermined sample characteristic may be an ion with a specific characteristic selected from one or more of mass, charge, mass-to-charge-ratio, fragment spectra, MS2 fragment information, ion mobility, intensity information, retention time, sequence (in case of proteins/peptides) or other identity (in case of lipids/metabolites). Optionally, the predetermined sample characteristic may be a plurality or a combination of ions. The predetermined sample characteristic may also be a sequence or structure information. Optionally, one or more sample characteristics may be used. Advantageously, mass, charge, mass-to-charge-ratio, fragment information, MS2 fragment information, ion mobility, intensity information, retention time, sequence (in case of proteins/peptides) or other identity (in case of lipids/metabolites) may be used directly and without a previous database search or de-novo interpretation of the data and can be directly used to generate a MS-integrity-code and for sample integrity analysis.

Optionally, the predetermined sample characteristic is one or more peptide(s) or protein(s), preferably quantified peptide(s) or protein(s). Advantageously, a quantified protein provides numerical values that can be used in the MS-integrity-code and that can be used to check the integrity of a sample. The numerical values of quantified peptides or proteins can be subjected to all mathematical calculations such as statistical and artificial intelligence methods, which use quantitative data, allowing the application of extensive data analysis methods. This is also advantageously for the generation of a calculable MS-integrity-code and to compare different samples to each other.

Optionally, the computer configured to process the mass spectrometry data to enhance the mass spectrometry data uses clustering algorithms to group samples based on their similarity. Here, many distances metrics can be used as a basis for similarity. As an example, distance can be defined as the difference in quantification of a defined protein subset. Additionally, different distance norms can be employed when using n-dimensional distances. Consider the case of using a Manhattan distance or Euclidean distance in a 2-dimensional case (e.g. quantification difference for two different protein subsets): When using Euclidean distance, a deviation in both protein subsets would give a smaller difference than when using Manhattan distance when comparing to having the same magnitude of distance on only one subset. Additional metrics arise from correlation, e.g. when calculating the Pearson correlation between two vectors. Before calculating sample similarity, samples may be normalized, scaled and aligned. Data can be clustered using state-of-the-art clustering algorithms, such as hierarchical clustering, density-based clustering and k-means clustering.

Optionally, the protein is selected from one or more of pregnancy zone protein (PZP), sex hormone binding globulin (SHBG), apolipoprotein(a) (LPA), other apolipoproteins (APOA1, APOB, APOA2, APOA4, APOC1, APOC3, APOC4, APOC2, APOD, APOE), immunoglobulin chains, hemoglobin subunits (HBA1, HBB, HBD, HBG1, HBE, HBZ), carbonic anhydrases (CA1, CA2), peroxiredoxins (PRDX2, PRDX6), catalase (CAT), band 3 anion transport protein (SLC4A1), spectrin chains (SPTA1, SPTB), ankyrin-1 (ANK1), glyceraldehyde-3-phosphate dehydrogenase (GAPDH), superoxide dismutase (SOD1), bisphosphoglycerate mutase (BPGM), actins (ACTB, ACTG1, ACTA1, ACTC1), selenium-binding protein 1 (SELENBP1), protein 4.1 (EPB41), L-lactate dehydrogenase B chain (LDHB), filamin-A (FLNA), talin-1 (TLN1), myosin-9 (MYH9), actin (ACTB), vinculin (VCL), alpha-actinin-1 (ACTN1), tropomyosin alpha-4 chain (TPM3), thrombospondin-1 (THBS1), thrombospondin-4 (THBS4), tubulins (TUBB1, TUBB4B,), 14-3-3 protein zeta/delta (YWHAZ), gelsolin (GSN), tubulin alpha-1B chain (TUBA1B), integrins (ITGA2B), coagulation factors (F13A1, F2, F5, F7, F9, F10, F11, F12), profilin-1 (PFN1), transgelin-2 (TAGLN2), fermitin family homolog 3 (FERMT3), RAS-related proteins (RAP1B), pleckstrin (PLECK) platelet basic protein (PPBP), fibrinogen chains (FGA, FGG, FGB), antithrombin-III (SERPINC1), prothrombin (F2), platelet glycoprotein Ib alpha chain (GP1BA), platelet factor 4 (PF4, PF4v1), extracellular matrix protein 1 (ECM1), clusterin (CLU), desmoplakin (DSP), WD repeat-containing protein 1 (WDR1), attractin (ATRN), platelet glycoprotein V (GP5), plasma serin protease inhibitor (SERPINA5), complement C1r subcomponent-like protein (C1RL), mannosyl-oligosaccharide 1,2-alpha-mannosidase IA (MAN1A1), kininogen-1 (KNG1), cholinesterase (BCHE), polymeric immunoglobulin receptor PIGR), keratins (KRT1, KRT10, KRT17, KRT2, KRT28, KRT9), fructose-bisphosphate aldolase (ALDOA, ALDOB), C-reactive protein (CRP), serum amyloid A proteins (SAA1, SAA2, SAA4), pregnancy specific pregnancy-specific beta-1-glycoprotein 1 (PSG1), pregnancy-specific beta-1-glycoprotein 9 (PSG9), actin-related protein 2 (ACTR2), prelaminA/C (LMNA), septin-9 (SEPTN9), peptidyl-prolyl cis-trans isomerase (FKBP2), V-type proton ATPase subunit B, brain isoform (ATP6V1B2). Optionally, the protein may be a protein isoform.

Preferably, the protein is selected from PZP, LPA, APOE, HBA1, FLNA, FGA, KRT9, CRP, PSG1, ACTR2. This is because PZP levels are suited to distinguish between female sex and male sex. LPA levels are highly individual specific. APOE is present in LDL particle and has an analysable genetic component. Quantified HBA1 is a marker for erythrocyte lysis, a sample collection and/or processing issue. Quantified FLNA and TLN1 are marker for platelet contamination, a sample collection and/or processing issue. Quantified FGA is an indicator for partial coagulation events, a sample collection and/or processing issue and FGA can further distinguish plasma and serum. High levels of KRT9 are an indicator for contamination of a sample by humans handling the sample. CRP levels can determine chronic inflammation. PSG1 is a protein strongly increased during pregnancy. ACTR2 is a protein increased in cell pellets obtained from a urine sample allowing to detect contamination of a urine sample.

Optionally, the predetermined sample characteristic is a quantified peptide. Advantageously, biological peptides are an additional class of molecules that can be analysed by MS. Moreover, cleaved peptides generated from proteins can be analysed without quantifying the proteins as a more direct measurement.

Optionally, the predetermined sample characteristic is a post-translational modification, preferably a quantified post translational modification. The post-translational modification may be a phosphorylation, glycosylation, glycation, ubiquitination, S-nitrosylation, methylation, N-acetylation, SUMOylation, and/or lipidation. Advantageously, post-translational modification can report on various biological features. Glycosylation can report on the biological activity of a distinct protein. Glycation can report on high blood glucose levels, present in diabetes. Phosphorylation can report on the activity of a protein as phosphorylation is one of the main proteins to activate signal cascades.

Optionally, the predetermined sample characteristic is an allele and/or variant peptide. Advantageously, mass spectrometry can also acquire genomic information. Alleles can be reflected on the protein level, if a DNA variant is translated in a protein variant with an alteration of the amino acid composition. Such protein variants can be used to identify a sample of a distinct individual and hence to check the integrity of a biological sample. Advantageously, such information might be already available from genomics or transcriptomics data of an individual or sample. In such cases no previous MS-measurement would be necessary to generate a MS-integrity-code. An additional advantage of the analysis of peptide variants is that an integrity check can be applied across different sample types. A MS-integrity-code may be generated and the allele combination of the proteins can be compared to any other protein containing sample such as muscle tissue.

Optionally, the allele is selected from one or more of the following genes listed by gene names: LPA, PON1, GC, APOB, APOE, AGT, A1BG, A2M, ABCC2, ACTB, ACTC1, ACTA1, ACTA2, ACTG2, ADIPOQ, AFM, AFP, AHNAK, AHSG, ALB, ALDH1A1, APOA4, APOH, APOL1, C3, HEL-S-62p, C4A, C7, CP, CPN2, F5, FGG; DKFZp779N0926, HBA1, HBB, HBD, HP, LBP, PGLYRP2, SERPINA1, SERPINF1, SERPINF2, F10, F11, F12, F13B, F2, F7, F9, SERPING1, TF, TTR;HEL111, ALDH1A3, ALDOA, ALDOB, AMBP, ANGPTL3, ANPEP, APCS, APEH, APMAP, APOA1, APOA2, APOC1, APOC3, APOC4, APOC4-APOC2, APOC2, APOD, APOF, APOM, ARHGAP1, ARSB, ATP1A4, ATP6V1A, ATRN, ATRNL1, AZGP1, B2M, BCHE, BLK, BLVRB, BTD, C15orf41, C1QA, C1QB, C1QC, C1R, C1RL, C1S, C2, C3, C4B, C4BPA, C4BPB, C5, C6, C8A, C8B, C8G, C9, CA1, CA2, CABIN1, CALD1, CALM1, CALM2, CALM3, CALR, CARD9, CARD11, CAT, CD14, CD163, CD44, CD5L, CDH5, CDHR2, CEP164, CFB, CFD, CFH, CFHR3, CFHR4, CFI, CFL1, CHGA, CHI3L1, CHIT1, CHRNB1, CKM, CLEC3B, CLTC, CLTCL1, CLU, CNDP1, CNTN3, COL18A1, COL6A3, COLEC11, COPE, CPB2, CPN1, CPS1, CRISP3, CRP, CRTAC1, CRYAB, CRYZ, CSH2, CSH1, CST3, CTSA, CTSD, CUBN, DBH, ECM1, EIF4A1, ENO1, ERLIN1, ERN1, ETFA, EXOC1, FABP4, FAH, FAM153A, FAM162A, FBLN1, FCGBP, FCGR3A, FCN2, FCN3, FETUB, FGA, FGB, FGFR2, FGG, FGL1, FITM1, FKBP4, FLII, FLOT2, FN1, GAPDH, GBA, GCA, GDI2, GGH, GLUD1, GLUD2, GP1BA, GPC6, GPLD1,GPRC5C, GPX3, GSN, GSTM4, HABP2, HADH, HARS, HBG2, HEXA, HGFAC, HIST1 H4A, HLA-A, HLA-H, HLA-C, HPR, HPX, HRG, HSP90AA1 ,HSP90B1, HSPA5, HSPA8, HSPG2, ICAM1, ICAM2, IGFALS, IGFBP3, IGFBP6, IL1RAP, INTS4, ITIH1, ITIH2, ITIH3, ITIH4, KCTD12, KIAA0319L, KLKB1, KNG1, KPNB1, KRT24, LAMB2, LCAT, LCN2, LCP1, LDHA, LDHB, LGALS3BP, LILRB1, LILRA1, LOC93432, LRG1, LRP2, LTF, LUM, LYVE1, LYZ, MANBA, MARCKS, MASP1, MASP2, MB, MBL2, MEI1, MIA3, MMP9, MMRN1, MPO, MST1, MST1L, MUC4, MYH11, MYH14, MYO1A, MYO1B, MYO1D, NCF4, NCKIPSD, NEO1, NIN, NRP2, ORM1, ORM2, PC, PCCA, PCDHA8, PCOLCE, PCYOX1, PDIA4, PEBP1, PF4V1, PF4, PFN1, PI16, PIGR, PLCD1, PLCG2, PLEC, PLG, PLS1, PLTP, PON3, PPA1, PPBP, PPIA, PPIL1, PRAP1, PRCC, PRDX2, PRG2, PRG4, PROC, PROCR, PROS1, PROZ, PRSS2, PSG1, PSMB1, PSMC6, PSMD2, PTGDS, PTPRF, PUS10, PZP, QSOX1, RAB21, RAN, RANBP2, RBP1, RBP4, RECK, REG1A, RNASE4, RNF111, RPL10, S100A9, SAA1, SAA2, SAA4, SDC1, SELL, SEPP1, SERPINA10, SERPINA3, SERPINA4, SERPINA5, SERPINA6, SERPINA7, SERPINB6, SERPINC1, SERPIND1, SFTPB, SHBG, SLC12A3,SLC3A2, SNCA, SOD3, SPP1, SPTA1, SPTAN1, SPTB, SRGN, STXBP5L, SUMO2, SUMO3, SUMO4, TAGLN2, TCP1, TFRC, TGFBI, THBS1, TIMP1, TMSB10, TMSB4X, TNC, TNXB, TOR3A, TRHDE, TTN, TTR, TXN, UBC, UBB, RPS27A, UBA52, UBBP4, UCHL3, UGT8, VASN, VCAM1, VNN1, VSIG4, VTN, VWF, YWHAE, ZNF256, ZNF652, preferably LPA, PON1, GC, APOB, APOE, AGT, A1BG, A2M, ABCC2, ACTB, ACTC1, ACTA1, ACTA2, ACTG2, ADIPOQ, AFM, AFP, AHNAK, AHSG, ALB, ALDH1A1, APOA4, APOH, APOL1, C3, HEL-S-62p, C4A, C7, CP, CPN2, F5, FGG, DKFZp779N0926, HBA1, HBB, HBD, HP, LBP, PGLYRP2, SERPINA1, SERPINF1, SERPINF2, F10, F11, F12, F13B, F2, F7, F9, SERPING1, TF, TTR, HEL111, ALDH1A3, ALDOA, ALDOB, AMBP, ANGPTL3, ANPEP, APCS, APEH, APMAP, APOA1, APOA2, more preferably LPA, PON1, GC, APOB, APOE, AGT, A1BG, A2M, ADIPOQ, AFM, ALB, APOA4, APOL1, C3, CP, CPN2, F5, FGG, more preferably LPA, PON1, GC, APOB, APOE, AGT, A1BG, A2M, ADIPOQ, AFM. Advantageously, the gene names of the list above relate to proteins that are highly abundant in plasma. As they are highly abundant in plasma, they are also common parts in other tissues as blood flows through all of our organs and can be commonly used across human and animal sample analysis. Additionally, peptide variants of the listed genes can be found in plasma.

Optionally, the predetermined sample characteristic comprises biomolecules such as lipids, small molecules such as drugs or metabolites, carbohydrates, preferably quantified biomolecules. Advantageously, mass spectrometry can identify and quantify other biomolecules and these have the advantage to supply an orthogonal level of biomolecule classes that can be used to generate a MS-integrity-code and to assess the integrity of a sample by a mass spectrometry system.

Optionally, the predetermined sample characteristic is an ion that are not specifically identified, and/or unidentified ions and/or raw data and/or other mass spectrometer acquired data. Advantageously, for such characteristics no extensive pre-processing is necessary to generate interpretable data to generate a MS-integrity-code and to assess the integrity of a sample.

Optionally, the predetermined sample characteristic comprises anthropometric data, e.g. height, weight, BMI of the origin of the sample. Advantageously, such data are often available for an individual and a MS-integrity-code can be generated without a MS measurement. A mass spectrometry analysis can be applied to assess the MS-integrity-code that is already pre-established by a anthropometric characteristic of the origin of the sample. For example, distinct biomolecules will correlate to a person's BMI. Another layer of parameters about an individual or sample that can be confirmed and compared to mass spectrometry analysis.

Optionally, the predetermined sample characteristic comprises medical relevant information such as clinical analysis, e.g. HDL, LDL, cholesterol, C-reactive protein (CRP), hemoglobin (Hb), red blood cell (RBC), white blood cell (WBC) count, lymphocyte count, neutrophil count, platelet count (PLTs), mean platelet volume (MPV), platelet distribution width (PDW), erythrocyte sedimentation rate (ESR)), information on health or disease state, genetic disorder and/or medication. Advantageously, such data are often available for an individual and a MS-integrity-code can be generated without a MS measurement. The mass spectrometry analysis can be applied to confirm the MS-integrity-code that is already pre-established by such a sample characteristic. For instance, clinical chemistry methods such as immunoassays can analyse biomolecules that are also quantified by a mass spectrometry system. Additionally, some parameters that are not directly measured in a clinical chemistry department will correlate with biomolecules quantified by a mass spectrometry system.

Optionally, the predetermined sample characteristic comprises information on sex, pregnancy, ethnicity. Advantageously, such data are often available for an individual and a MS-integrity-code can be generated without a MS analysis. The mass spectrometry analysis can be applied to confirm the MS-integrity-code that is already pre-established by such a sample characteristic. For example, the levels of distinct proteins such as PSG1 will indicate pregnancy and other proteins such as PZP will indicate sex.

Optionally, the predetermined sample characteristic is information on metadata about an individual such as from transcriptomics, genomics or metabolomics analysis. Advantageously, such data are often available for an individual and a MS-integrity-code can be generated without a MS analysis. The mass spectrometry analysis can be applied to confirm the MS-integrity-code that is already pre-established by such a sample characteristic.

According to a third aspect of the invention, there is provided a computer program for controlling any of the methods described above.

According to a fourth aspect of the invention, there is provided a computer-readable medium comprising instructions for controlling any of the methods described above.

According to a fifth aspect of the invention, there is provided a computer system for processing mass spectrometry data, the computer system configured to process the mass spectrometry data to determine a sample characteristic of the biological sample. The computer system for processing mass spectrometry data may generate a similarity metrics between a sample analysed by a mass spectrometry system and a predetermined sample characteristic from a sample analysed by mass spectrometry and/or another non-mass spectrometry generated sample characteristic; and output an indication of the integrity of the biological sample.

According to a sixth aspect of the invention, there is provided a use of mass spectrometry data to assess the integrity of a biological sample. Optionally, the use may comprise one or more of the following use(s): to track a sample, to determine the biological origin of the sample, to detect alterations of the MS-integrity-code by contamination with another sample, to detect alterations of the MS-integrity-code by problems with sample processing e.g. erythrocyte lysis, sample storage problems, problems in the MS-workflow, to match information to a sample, to the matching of one or multiple sample to a distinct person, to the matching of one or multiple samples to each other, to match of one or multiple samples to distinct clinical parameter, to identify an individual, to determine the similarity of two or more samples, to determine the similarity of two or more individuals, to allow to make a similarity determination between samples of one individual but also between individuals, to exclude a similarity of two or more samples, to determine a dissimilarity between two or more samples, to determine a dissimilarity of two or more individuals, and/or to determine if two samples are from the same tissue origin e.g. if two times plasma is analysed and not once plasma and once urine.

According to a seventh aspect of the invention, there is provided a machine-readable code such as a matrix code (e.g. a barcode and/or a QR code). The machine-readable code encodes a predetermined sample characteristic such as a protein level, allele information, sample contamination indicator, information on sex and is a machine-readable code reflecting directly the MS-integrity-code. Preferably, the machine-readable code is a physical label, for example a sticker for attaching to a sample container and/ortube. Optionally, the machine-readable code is for use in identifying the sample and/or its origin. Advantageously, the physical code attached to a sample container delivers directly machine-readable information physically linked to the sample. A mass spectrometry system may generate integrity data, which can be compared with a sample characteristic in the physical code. Advantageously, this allows for confirmation if a suspected sample is in the labelled container.

According to an eighth aspect of the invention, there is provided a use of a predetermined sample characteristic and a mass spectrometry system configured to provide mass spectrometer data, wherein the use is in cryptography. Using a predetermined sample characteristic in a MS-integrity-code can be applied for providing encrypted handling of information and a mass spectrometry system providing mass spectrometer data can be applied to decrypt the information. Advantageously, the key to decrypt the information is provided from a sample of the same biological origin/individual of which the predetermined sample characteristic has be used for the MS-integrity-code. Advantageously, this will allow an individual specific cryptography process. Advantageously, this allows creating a secure sample analysis platform, where only correctly acquired sample data can be used to correctly decrypt a code based on a predetermined sample characteristic in the MS-integrity-code. Moreover, such a setting might be directly applied not only for having a secured sample analysis platform, but also as a key that gives access to health relevant information of a patient.

Optionally, the use may be a use of a predetermined sample characteristic for encrypting data to provide encrypted data and a mass spectrometry system configured to provide mass spectrometer data for decrypting the encrypted data.

Optionally, a chemical modification can be used to generate a specific signature to the MS-integrity-code. Advantageously, chemical modifications can be added to biomolecules in a mass spectrometer sample preparation workflow, allowing further variation of the biomolecules. Advantageously, specific signatures may be added that can be linked to a specific additional information e.g. a laboratory specific signature.

Optionally, using a MS-integrity-code in cryptography may be applied to encrypt data in a healthcare system.

### List of Figures

Embodiments of the invention will now be described, by way of example only, with reference to the accompanying figures, in which:
Figure 1 is a schematic overview of predetermined sample characteristics that can be integrated in a MS-integrity-code (A) and the application of a mass spectrometry system in combination with the MS-integrity-code to assess the integrity of a sample (B).

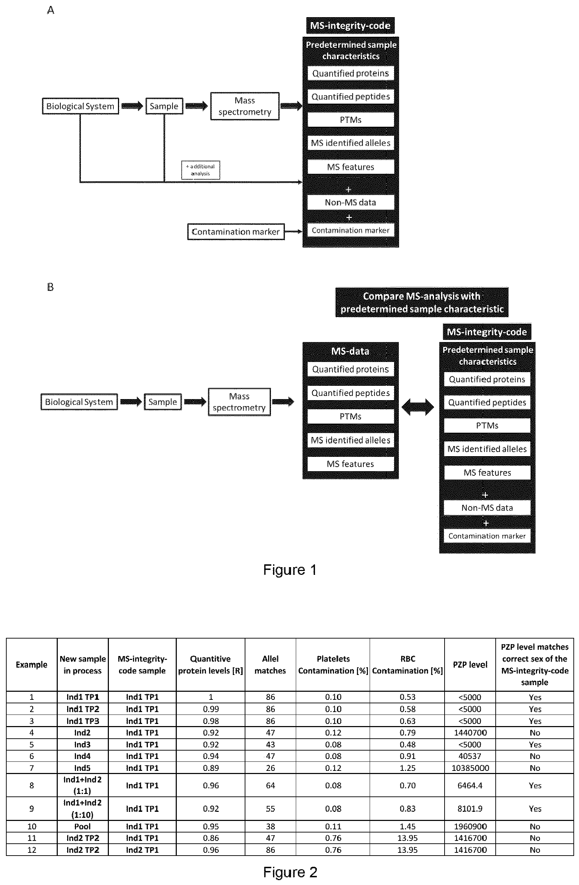

Figure 2 is an overview table of examples for the comparison of predetermined sample characteristics in a MS-integrity-code and data generated by a mass spectrometry system to evaluate the sample integrity. The "MS-integrity-code sample" is the sample of the biological system of which the predetermined sample characteristic for the MS-integrity-code was established. The "New sample in process" is the sample of which a mass spectrometry system determined data for the comparison with the data of the predetermined sample characteristic. The comparisons across different sample characteristics are shown.
Figure 3 is a summary table containing MS-integrity-code examples and their comparison with data generated by a mass spectrometry system, resulting in examples of similarity metrics that are used for the assessment of the sample integrity.
Figure 4 is an example of a predetermined sample characteristics in a MS-integrity-code (A) that were translated in a machine readable QR code (B).
Figure 5 is a scatter plot showing pairwise comparison of protein levels in samples from one or more individual at one or more time points.
Figure 6 is allele information in a MS-integrity-code for assessing the origin of a sample, in which (A) summarises the comparison of samples from 5 individuals at three different time points; and (B) graphically shows the determination of similarity with a hierarchical clustering in a plot.
Figure 7 is a plot showing the application of quantitative MS-generated values from samples as a similarity index to track samples by using quantitative protein levels (A), quantitative peptide levels (B) and quantitative post-translational modification levels (C).
Figure 8 is a series of scatter plots which show in Figure 8A the correlation of Individual 1 at time point 1 vs. Individual 1 at time point 2; in Figure 8B the correlation of plasma samples from Individual 1 *vs.* a 1:1 dilution of plasma samples from Individual 1 and Individual 2; and in Figure 8C the correlation of plasma samples from Individual 1 *vs.* a 1:10 dilution of plasma samples from Individual 1 and Individual 2.
Figure 9 is a scatter plot comparing quantitative protein levels in plasma samples from Individual 2 time point 2 vs. Individual 2 time point 1, with a series of highlighted proteins that are increased levels of contaminants.

### Detailed description

Mass spectrometry (MS) may be used to verify the integrity of a biological sample. The method may apply mass spectrometry analyses to use measured ions, the quantification of ions, identified and quantified proteins and peptides, identified alleles from peptides or proteins and identified and quantified metabolites or lipids for this purpose. Tracking of a sample and confirming that a distinct analysed sample is the intended sample is of utmost importance for example in studies, biobanking and clinical diagnostics. A mass spectrometer may measure the mass-to-charge-ratio of ions. These ions may be peptides, proteins, modified variants of peptides or proteins, unidentifiable ions, but also metabolites or lipids. The presence or absence of these factors may result in a pattern that may be used as a barcode or fingerprint of a biological sample which we define as a code that can be applied to confirm the integrity of the sample - a "MS-integrity-code". The information of such a MS-integrity-code is usually one or more predetermined characteristics of a biological system or a sample. Herein, the sample may be any kind of biological sample for example blood, plasma, serum, urine or tissue such as muscle or tumour tissue or biopsies, including liquid biopsies. The sample may be a processed sample, e.g. dried blood, plasma, serum, depleted plasma and exosomes from plasma or other biofluids. A mass spectrometry system can retrieve information of biomolecules that can be deterministic for an individual as each individual has its own specific lifestyle and life history, which can be very different for different individuals. This will result in distinct levels of biomolecules in a biological sample such as plasma. Information on single or multiple of such biomolecules can be applied to generate a MS-integrity-code for a biological system. If a sample with the same origin (e.g. from the same donor) e.g. the same biological system is measured by a mass spectrometry system or if two samples with the same origin are analysed together, a similarity metrics can be generated and used to confirm the integrity of a sample, including tracking of a sample, identifying the origin of a sample and assessing the quality of the sample The biological system may be a human. The biological system may be an animal, e.g. cat, dog, laboratory animals (mouse, rat, rabbit, frog, fish), horse, camel, and/or fish. Moreover, MS-based analyses can identify single amino acid polymorphisms (SAPs) in the form of peptide and protein variants. These analyses can be performed for all kinds of genetic variations that are translated to a peptide or protein sequence such as human single nucleotide variations, microsatellites, and small-scale insertions and deletions. The identified peptide and protein sequence variations can be applied to track a sample and assign it to a distinct origin. Moreover, this information can be applied to analyse genetic diversity and distance as well as the origin of a sample and the quality or contamination of a sample such as human blood sample with one or more samples of other individuals. To achieve the identification of peptides one can use mass spectrometry such as a sample preparation workflow, liquid chromatography coupled to a mass spectrometer and an ionization technique such as electrospray to produce ionized biomolecules that can be analysed by a mass spectrometer and a computer system for performing processing tasks of acquired data. In MS-based proteomics peptides are produced by isolating non-digested peptides or enzymatic cleavages of peptides bonds by a protease such as trypsin alone or in combination with any other protease or chemical or physical method or the use of other proteases, chemical or physical methods without alone or in combination without the protease trypsin.
Figure 1 summarises the generation and application of a mass spectrometry system to determine the integrity of a biological sample that is harvested from a biological system, for example a blood sample originating from a human individual. A mass spectrometry system can analyse a plethora of biomolecules. These biomolecules can provide intelligence on a distinct sample characteristic such as anthropometric data, clinical chemistry analysis, information on sex, age, disease status, medication, metadata such as sequencing data (called non-MS data in Figure 1A) and of course also on a mass spectrometer determined biomolecule (Figure 1A). Such sample characteristic can be predetermined for a distinct biological system and linked to this biological system and a sample generated from this biological system. If a sample is obtained again from this biological system, a mass spectrometry system can be used to generate mass spectrometry data of the biological sample. This information can be used to assess the integrity of the sample. The mass spectrometry data can be processed and compared to a predetermined sample characteristic to retrieve an indication of the integrity of a sample (Figure 1B). The predetermined sample characteristic to which the newly acquired mass spectrometry data is compared can be seen as an integrity-code, called a MS-integrity-code. Wherein one or more predetermined sample characteristics can be implemented in the MS-integrity-code.
In a specific example, electronic health data of an individual are stored in a database. This information might contain previous clinical chemistry measurements, information on sex, genetic information and potential diseases. Such predetermined sample characteristics are linked to the individual and can be used to generate a MS-integrity-code. In a future contact with the healthcare system, a new blood sample is taken for analysis from this individual. A mass spectrometry system can be used to assess the sample integrity. The mass spectrometry system will generate mass spectrometry data that provide intelligence about a sample characteristic that were predetermined and are part of the MS-integrity-code. In this example, the mass spectrometry data can confirm the integrity. It can confirm that the sample is taken from the correct individual linked in the healthcare system by calculating a similarity between one or more predetermined sample characteristics and newly acquired MS information. In this example, distinct predetermined protein levels can be compared with newly acquired protein levels, predetermined genetic information can be compared with newly MS-acquired allelic information, sex can be confirmed by sex-related information and the integrity in terms of sample processing issues can be assessed by distinct contamination markers. In this way, the integrity of the sample can be evaluated.
In the scenario, that all future plasma and other samples in the healthcare system are analysed by mass spectrometry one would like to establish databases including MS-integrity-codes based on a predetermined sample characteristic. So once a person is measured for the first time her/his information will be added to the database and linked to this person. This will allow the doctor or a tracking system to cross-check if the sample integrity is intact.

MS-based analyses can quantify a large number of biomolecules of different types in any biological system. Many biomolecules can be quantified within one analysis in human blood plasma sample or any other kind of liquid biopsy or tissue sample. The high number of quantified biomolecules per sample allows to generate a MS-integrity-integrity-code that is specific for this sample. If another sample of the identical biological system is taken and analysed by an MS-based analysis, the two samples can be compared by the many quantified biomolecules which would allow to make a conclusion of their shared origin. This includes the presence or absence of one or more distinct biomolecules. It will also include the quantity, presence or absence of peptides, alleles that are determined by mass spectrometry, post translational information or measured ion patterns or biomolecules with a recorded MS2 fragment spectrum which can presently not identified as a specific known biomolecule. Each of this information or their combination results in a pattern that is unique for the origin of a sample. This pattern can be interpreted as an intrinsic or endogenous barcode or MS-integrity-code for a sample. It will allow to determine the origin of the sample, the similarity of two samples and also if a sample is contaminated with one or more other samples or if the processing of a sample was incorrect.

We herein propose a method that allows to use a mass spectrometry system to confirm the integrity of a sample and to generate a sample intrinsic code that reflects the integrity of a sample. The method can generate and read such MS-integrity-codes from biological sample to track sample, to re-identify sample when their label is lost, to match sample to each other or exclude a shared origin of two or more sample, to validate the correct labelling. This code can be compared to theoretical codes generated with other methods and information than mass spectrometry and a MS can be used to compare the codes and confirm the integrity. As we propose herein an integrity check of a sample, this "MS-integrity-code" can also be used to detect the contamination of a sample e.g. with one or more other samples.

Tracking of a sample is particularly advantageous if a sample has been measured already or if two samples with the same origin (e.g. from the same individual) will be analysed e.g. in a longitudinal study. However, it will be also advantageous if information about relatives of the individual is known as the MS-integrity-code will allow to make a relation between a sample and its origin. Moreover, the data acquired during the performance of an MS-based analysis will allow us to evaluate the sample origin with other available metadata such as anthropometric data, information about sex or ethical origin. The acquired data will also allow to check for a contamination of one or more samples.

In clinical practice and in studies analysing human (or animal) samples, such as in epidemiological or biomarker discovery studies, it is of utmost importance that the correct (clinical) phenotype is matched with the correct sample. For example, this will allow that the analysed parameter e.g. in a clinical chemistry department is delivered back to the right patient or her/his physician. However, accidental or deliberately swapping of a sample can happen. In a clinical setting, miss-assigned test results to patients will result in making incorrect clinical decisions and in a study analysing human samples this will lead to spurious study results. If a sample has been measured by MS-based analysis before or if information about the alleles is available e.g. from genomics or transcriptomics analysis, it is possible to use allele information obtained by MS-based proteomics to assign a sample to the matching individual. This will allow that other metadata can be assigned to the correct person in a study setting and it will allow that the diagnostic results of a person is transferred to the correct person, ultimately erasing sample swapping errors.

The method of the invention may track the identity of a sample throughout processes and analysis. Longitudinal sampled plasma may be analysed with regards to the alleles in each sample and to evaluate if two samples are really originating from the same individual. With continuous technological progress, it is likely that mass spectrometry enters clinical medicine on a broad scale. A MS-integrity-code can be generated for each individual. The information saved in biomolecules analysed by MS can be applied to confirm the identity of a sample or to map the sample to a specific origin.

Similarly, under distinct conditions mixing or cross-contamination of a sample might occur. For instance, in a clinical study it might be necessary to combine two or more aliquots of the same sample. In such cases it might happen that one aliquot is combined with another wrong sample. Allele information can be used to determine the presence of different alleles in one sample. We provide evidence for this possibility by mixing two samples of two different individuals of which the genotype of the one individual was known before. This allowed us to detect allele that should not be present in the sample of the individual that should be analysed.

A biological sample is analysed by mass spectrometry to obtain amino acid sequences of peptides and proteins in the sample. As the technology MS-based proteomics can determine the sequence of a peptide, we can also identify SAPs caused by coding SNPs or individual specific allele variations by the peptide sequences. Usually publicly available databases can be applied to match the experimentally determined mass spectra from the MS analysis to a predicted spectrum from databases and calculate the likelihood that a distinct sequence is present. For this purpose, publicly available databases such as UniProt are usually applied. However, these databases usually contain only one allele which is often the most common allele. Hence, variant peptides containing SAPs or other genetic alterations reflected on the amino acid sequence of a protein cannot generally be detected by standard proteomics analysis, because they are absence in the employed generic databases for the mass spectra interpretation. However, one can create custom databases with variant sequences which is for example possible with the Swissknife PERL module from UniProt or use the publicly available database dbSAP. The generated variant FASTA files allow now the database search for variant sequences. This is possible by using such tools or by creating them with other tools or manually. Large-scale detection of hundreds of alleles by detecting sequence specific alleles by MS-based proteomics from body fluids and tissues is possible when using such variant databases, which can be applied for disease diagnosis and identification of an individual sample and contamination of a sample from other sources. The method may be used to determine the variant sequences by de novo sequencing of peptides for which no database is necessary. Once the peptide or protein sequence is obtained, various analyses can be subsequently performed. The presence or absence of a protein variant can be determined. This will also allow the determination of whether a gene locus is homozygous or heterozygous, as most organisms are not haploid and thus have more than one allele of each gene locus (e.g. humans are diploid and have two alleles of each gene locus apart from the sex chromosomes). The data can be applied to the analysis of ethnic origin and for the determination of population frequencies of protein variants. In general, the MS-based analysis of protein variants can be pursued in all fields and for all applications where genomics and transcriptomics are used. One bottleneck of the identification of alleles by MS-based proteomics is that not all peptides are equally well suited for analysis. Some parts of a protein might not result in analysed peptides. Moreover, in distinct mass spectrometer data acquisition or scan modes, the sequencing of peptides with MS2 spectra depends on a semi-stochastic process such as in data dependent acquisition (DDA) and partially also in data independent acquisition (DIA) modes. Therefore, it is possible that distinct peptides will not be detected by MS at all or some others are missing in distinct situations. However, the MS technology is improving constantly, and the gained spectra information are becoming increasingly complete. Moreover, novel scan modes such as targeted acquisition of predefined peptides will result in the acquisition of distinct peptides with a very high probability. Such targeted methods can result in as high number of analysed peptides as the untargeted shot-gun methods such as DDA and DIA. Additionally, the likelihood for peptide identification can be increased by using further information or prediction generated from experimental data. For example, it is possible to predict the detectability of peptides by analysing large datasets by using machine learning tools and by predicting the detectability of peptides. The abundance of other peptides of the same protein or even of other proteins can be used to further increase the likelihood that distinct peptides are correctly identified. This becomes especially helpful if the database search space is increasing e.g. by using a database with peptide variants. Moreover, if allele information and variant peptides are used in the MS-integrity-code to match a sample the missing value problem can be considered in the calculations for the matching likelihood. For example, an identified variant peptide could get a higher score than a non-present variant peptide.

MS-based analyses can quantify a large number of biomolecules in any biological system. Many proteins can be quantified in only one analysis of a human blood plasma sample or any other kind of liquid biopsy or tissue sample. The high number of quantified biomolecules per sample allows the generation of a MS-integrity-code that is specific for this sample. If another sample of the same biological system is taken and analysed by MS-based analyses, the two samples can be compared based on the many quantified proteins which would allow drawing a conclusion of their shared origin. This includes the presence, absence or difference in abundance of one or more distinct biomolecules. In case the biomolecules analysed are proteins and peptides, it will also include the quantity, presence or absence of peptides, alleles that are determined by mass spectrometry, PTM, measured ion patterns or "features". Each of this information or the combination of information results in a pattern that is unique for the origin of a sample. This pattern can be interpreted as an intrinsic or endogenous label or in other words an MS-integrity-code for a sample. It will allow the determination of the origin of the sample, the similarity of two samples and also whether a sample is contaminated with one or more other samples or if the processing of a sample was incorrect.

### Examples

The integrity of a sample can for example be obscured due to various reasons including
(A) unknown or corrupted origin of a sample
(B) losing the identity of a sample
(C) potential contamination of a sample
(D) problems in sample processing
(E) degradation of a sample

Here, we describe in real experiments the generation of a MS-integrity-code with predetermined sample characteristics for plasma samples and how a mass spectrometry system can be applied to confirm the integrity of the sample. For this purpose, we harvested plasma samples of five individuals (Ind1, Ind2, Ind3, Ind4, Ind5) at three different time points (TP1, TP2, TP3). We analysed the samples with a mass spectrometry system to generate information on proteins, peptides, PTMs and alleles.

The MS-integrity-code can consist of all kinds of predetermined sample characteristics e.g. quantitative MS-derived data, allelic information determined by MS and other available information that can be used to check the integrity of a sample such as sex or genetic information. Here, we used mass spectrometry quantified protein levels, sequencing identified alleles, indicators for the sample quality (platelet and erythrocyte contamination marker intensities) and sex as a predetermined sample characteristic (Figure 2). These predetermined sample characteristics can serve as a MS-integrity-code and can be connected to a biological system, an individual or a sample. If a sample of a biological system with a predetermined sample characteristic is analysed by a mass spectrometry system, the mass spectrometry system will provide mass spectrometry data of the sample and the mass spectrometry data can be processed to determine a value of the predetermined sample characteristic. The comparison of the newly acquired MS data and the information of the predetermined sample characteristic allows to calculate a similarity value.

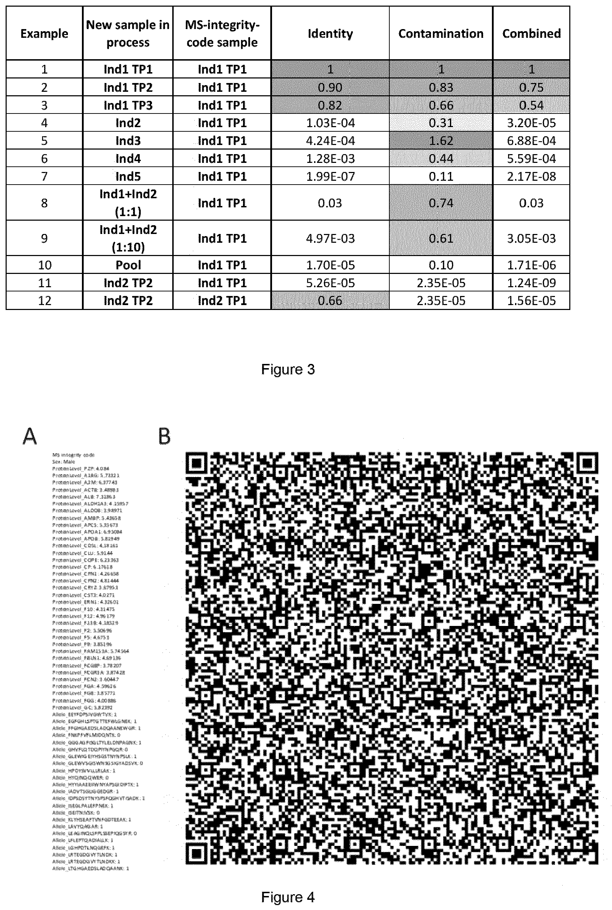

The determined similarity allows to confirm the sample integrity using for example a scoring system/function (Figure 3). We supply various application examples for integrity checks using one or more predetermined sample characteristics and diverse data types (Figure 2, Figure 3).

Similarity may be generally defined as the distance of two samples, based on predetermined distance metrics. Examples include Manhattan distance (e.g. sum of differences), Euclidean distance (e.g. shortest distance (Pythagorean distance) or correlation distance (e.g. the correlation of vectors). Overlap may be defined by fixed overlap thresholds (e.g. R>= 0.95) or may be estimated from given sample deviations (e.g. measure the same sample repeatedly and use the calculated sample-to-sample correlation as a threshold).

### Example 1 - Identity of a sample

One possibility to obscure the integrity of a sample is to lose or change its identity. In an experiment we compared a sample to itself. For the sample, we predetermined sample characteristics, including quantitative protein levels, alleles, levels of sample quality indicators and sex of the individual of which the sample has been obtained (Figure 2). These predetermined sample characteristics can be used as a MS-integrity-code of a distinct individual. Here, we want to check the integrity of this sample again. We use a mass spectrometry system configured to provide mass spectrometry data of the sample and process the mass spectrometry data to determine a sample characteristic that was also predetermined and are part of the MS-integrity-code. The comparison of the newly acquired MS data and the information of the predetermined sample characteristic allows the calculation of a similarity index of the samples. This resulted in the highest possible similarity with regards to the assessed parameters (Example 1, Figure 2). The quantitative protein levels were perfectly overlapping (Pearson R=1), the predefined alleles were identical with the mass spectrometric determined alleles (86 allele matches) and the predefined sex (male) was reflected by the low PZP levels. Moreover, it is possible to calculate overall scores for identity, contamination and the combination of identity and contamination, which were high in this example (Example 1, Figure 3). Such scores for the similarity of the MS-integrity-code predetermined sample characteristics and newly acquired MS information is displayed in Figure 3 for various examples.

However, the composition of biomolecules within a sample can change over time due to differences in the measurement methods. Nevertheless, the information obtained from the same person over time will remain more similar to each other than the comparison to a sample of another person. From the same individual two more samples at different time points have been harvested and measured by MS. The predetermined sample characteristics in the MS-integrity-code have been compared to the MS data, resulting in a high similarity of quantitative protein levels, alleles and sex-related proteins (Example 2 and 3, Figure 2). Thus, we could confirm the integrity of these samples. This is possible using the individual data types for which MS-readouts are available, but we also used non-MS data such as information about sex and alleles as a predetermined sample characteristic.

Next, we compared the MS-integrity-code of one individual to a sample of another individual to show the possibility of identifying corruption of the integrity of a sample. For this purpose, we analysed additional samples. The quantitative MS-derived information on quantitative protein levels, PZP levels and alleles were compared to the predetermined sample characteristics in the MS-integrity-code. In each example, the outcome was that integrity was corrupted, because predetermined sample characteristics of the MS-integrity-code were obtained from a different individual than the samples of which the mass spectrometry system provided information (Examples 4, 5, 6, 7; Figure 2 and 3). Moreover, in the examples 4 and 7 (individuals 2 and 5), the sex-related protein PZP suggested women as the donors of the samples. However, in the MS-integrity-code, we stored the information that our sample was obtained from a male individual. Female sex was determined by the very high PZP levels, which alone would have been enough information to confirm that the integrity with regards to sample identity was corrupted. Combining the single data from Figure 2 into scores for identity, contamination and the complete MS-integrity-score, instantly highlighted the significantly low scores for these examples with regards to the identity and therefore also the complete MS-integrity-score. In these examples the scoring function was designed that even small differences between the predetermined sample characteristic in the MS-integrity-code and the MS-acquired information result in a strong signal. For example, comparing two samples of the same individual resulted in a perfect MS-integrity-score of 1 for the same sample or 0.75 for a sample at a different time point (Example 1 and 2; Figure 3). However, comparing the MS-integrity-code to a sample from a different individual resulted in scores as low as 2.17E-08 (Example 7; Figure 3). This will solve the problem (A) of having an unknown or corrupted origin of a sample. Moreover, this will allow to re-link a sample to its origin even if the sample labels would be lost are mixed. The correct plasma sample (and its newly obtained information) can be linked to its origin by calculating a similarity between the newly acquired mass spectrometry data and the predetermined sample characteristic in the MS-integrity-code. In this case, the similarity is reflected by the MS-integrity-scores for the examples 3, 4, 5, 6 and 7. Only the example sample 3 resulted in a high MS-integrity-score (Figure 3). This will solve the problem (A) of losing the identity of a sample.

The predetermined sample characteristic in the MS-integrity-code can also be directly translated in a machine-readable code such as a matrix code (barcode or a QR code). Such a barcode would directly contain readable information of the predetermined sample characteristic. These data can be read with a barcode scanner and easily compared with mass spectrometry system acquired integrity data. We exemplified the translation of a MS-integrity-code that consisted of information based on sex, protein levels, allele information and protein levels of contamination indicators such as fibrinogen (FGA) (Figure 4A) and translated this information in a QR code (Figure 4B).

To further explain the MS-acquired data and how they can be used for checking the integrity of a sample with regards to their identity, we will explain in the following paragraph the individual data in more detail.

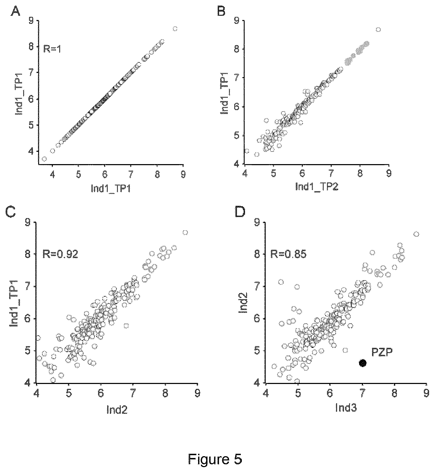

Figure 5 demonstrates pairwise comparison of protein levels in samples. Combining the quantitative information of all analysed biomolecules between samples is particularly valuable for this purpose. As an example, the plasma proteome of a sample is ranging over ten orders of magnitude from the highest to the lowest abundant proteins. A mass spectrometry system can detect about 5-6 orders of magnitude of the plasma proteome today (Figure 5A). Quantified proteins in blood plasma of one sample "Ind1" at one time point "TP1" correlating to the same sample to illustrate the covered abundance range of quantified proteins. The x-axis displays the intensity level for one sample and the y-axis the intensity for a second sample. These quantitative proteomes can be stored in a databank and contain the quantitative information of hundreds of proteins. As protein levels are specific to an individual, they do not change dramatically overtime (with the exception of protein changes under distinct circumstances e.g. disease). Comparing the whole quantitative proteomes of samples of the same individual over time results in similar quantitative proteomes for example with a Pearson correlation coefficient of 0.99 (Figure 5B). The similarity between the levels of proteins of two different individual will be lower e.g. overall Pearson correlation coefficient of 0.92 (Figure 5C). Moreover, the level of distinct other proteins can be prominently different such as the sex hormone binding globulin (SHBG) or the pregnancy zone protein (PZP) that can be 10 to 100-fold different between women and men and increase another 10 to 100-fold during pregnancy (Figure 5D). As such, the information of the sex can be used in the MS-integrity-code. Another example of highly informative proteins that are strongly different between individuals is the apolipoprotein(a) that can display thousand-fold difference in expression between individuals. These quantitative protein values can mainly be used to validate the integrity of a sample with respect to its identity or that the sample has not been exchanged or corrupted in a process. Such highly individual-specific protein levels have an especially high value for distinguishing the origin of a sample. As the value of proteins and other factors quantified by mass spectrometry have different values to distinguish individuals, a weighting or scoring function can be applied where such proteins would be more important to determine the sample origin. Moreover, proteins influenced by distinct life situations can be given a lower importance or such life situations can be considered otherwise in a scoring function. An example would be the inflammation protein SAA1, which can be seen as individual-specific, but which can also vary several hundred-fold upon infection. Additionally, especially when it comes to identifiability a stepwise process can be applied. For example, first the sex could be determined by sex-dependent protein levels, next potential sample donors can be narrowed down by considering a strongly individual-specific protein such as apolipoprotein(a) and/or excluding an individual as the allele composition does not match. The quantitative protein levels will further allow to confirm the integrity of the sample with regards to its sample type e.g. that a plasma sample is a plasma sample and not a serum sample, a sample from another animal or another body fluid. It will also inform, if the sample has been degraded.

Next to quantitative MS-derived data about the levels of proteins, peptides and PTMs, a mass spectrometry can identify alleles. These will be of high valuable to check the integrity of a sample throughout a process. Information about the absence or presence of MS-identified alleles are complementary to the quantitative information and strengthen the MS-integrity-code.

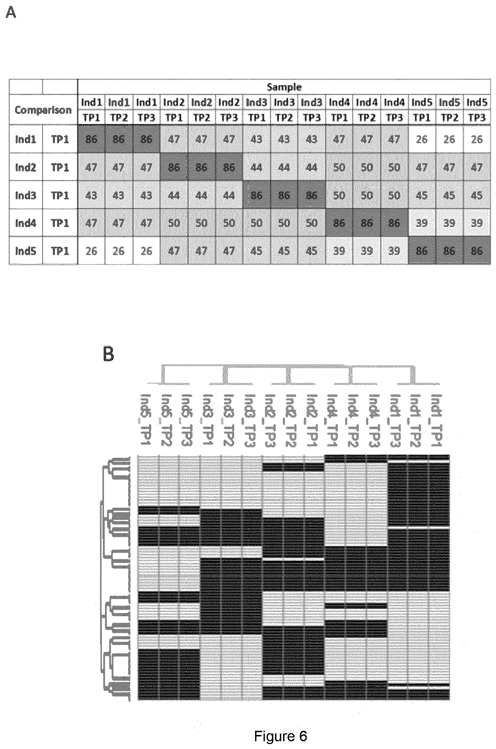

Figure 6 shows allele information in a MS-integrity-code for sample tracking or identification of the origin of a sample. To exemplify that such information can be used to check the sample integrity with regards to its identity, we took blood and harvested plasma from five individuals (Ind1, Ind2, Ind3, Ind4, Ind5) at three different time points (TP1, TP2, TP3). The plasma samples were prepared and analysed by MS-based proteomics to quantify proteins. Allele information can be applied for this purpose. The samples collected at the first time point have been used as a MS-integrity-code for each of the five individuals.

We asked the question if it would be possible to track a sample, if a MS-integrity-code based on genetic information would be available. For this purpose, we assumed that the genetic information of the sample of the first time point TP1 was known. We used for this purpose the mass spectrometry acquired spectra and interpreted them with a variant FASTA file that contained information on SAPs. Allele information or information about mutations may also be used that have may been acquired by other methods such as genomics or transcriptomics. Next, we wondered whether it is possible to correctly assign the samples of the two other time points (TP2, TP3) to the first time point (TP1). The predetermined sample characteristics are the information on the alleles and are part of the MS-integrity-code. These were compared at all three time points. In all binary comparison always the samples from the same individual resulted in the highest possible overlap of alleles from different genes. The total number of alleles from different genes was 86. The number of overlapping alleles in the binary comparisons is color-coded from low (white) to high (grey) overlapping numbers of alleles (Figure 6A). Figure 6B shows the same data, but we used a hierarchical clustering algorithm to sort the samples automatically into groups of similar samples. In each case the samples were correctly grouped together. This confirms that it is possible to use MS-acquired genetic information confirm the integrity of the sample. This solves (at least) the problems (A) of unknown or corrupted origin of a sample, (B) losing the identity of a sample and (C) to detect potential contamination of a sample as set out above. The number of alleles may be much higher in other tissues and can be further filtered to just a distinct subset. The advantages derived from the dataset can be further improved by refining the data. For example, the first three orders of magnitude of MS-quantified peptides will be more reproducible detected due to the so called missing-value-problem in proteomics. Therefore, it will be more valuable to just use peptides that are relatively high abundant or that are quantified by targeted or DIA methods.

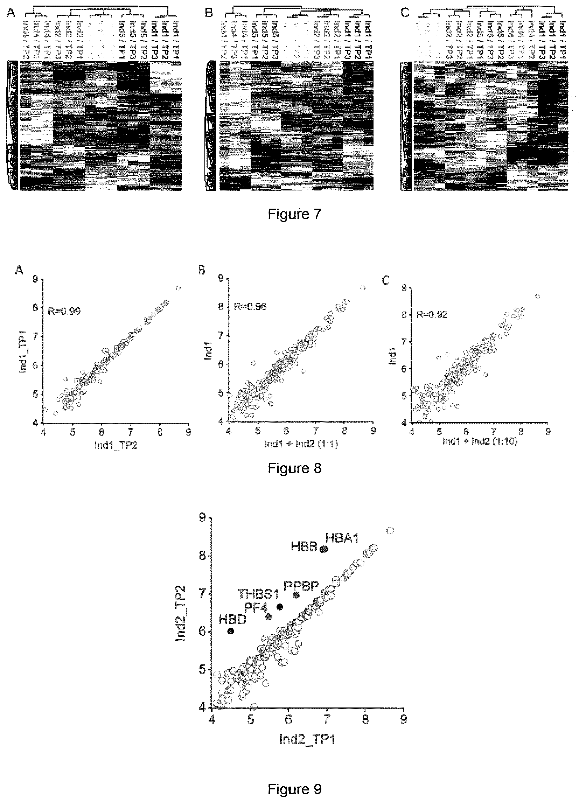

Next, we wanted to illustrate that also single information of the MS-integrity-code, such as the quantitative information alone can be used to check the integrity of a sample. Figure 7 shows the application of quantitative MS-generated values from biological samples as a similarity index to determine the origin of samples. We took blood plasma of five individuals (Ind1, Ind2, Ind3, Ind4, Ind5) at three time points (TP1, TP2, TP3) and acquired MS-based proteomics data to illustrate the determination of a shared origin of two or more samples. Quantitative proteomes of the MS-based analysed samples included several hundreds of quantified proteins. A hierarchical clustering algorithm grouped automatically and in an unbiased fashion, the samples of the same individual consistently together (Figure 7A) The five individuals are abbreviated with "Ind1-5" and the time points with "TP1-3". Black means high expression values and white low expression values for a distinct protein, respectively. The hierarchical clustering assigned always the three samples of the same individual to each other. This would allow us to track samples in our workflow as we can elucidate that two or more samples have the same origin. In a typical application case, we could have information on a previously acquired proteome of the same individual e.g. in a database. If we would measure now an additional sample, we can check if this sample is really from the same person or from a different person. This would also allow us to match metadata to this sample. Of note, manual interpretation or interpretation with other methods than hierarchical clustering that allow the calculation of a similarity distance between two or more samples would be possible as well - for example machine learning algorithms. Thus, it is possible to use quantitative MS-derived information as a part of a MS-integrity-code to track or match samples.

Next to the quantity of proteins, one can also use other information acquired by a mass spectrometer. To illustrate this, we used peptide information and post-translational modifications (Figure 7B, C). Next, we applied a hierarchical clustering algorithm and were able to assign the three samples with the shared origin to each other, again allowing tracking of the samples.

Pre-processing of the data can further increase the clustering. Possibilities could be to use just the first three orders of quantified proteins as they are usually more reproducible quantified. Another possibility would be to use individual-specific protein levels, which are the levels of proteins that have shown in large-scale studies to be very constant over a long time period.

This solves (at least) the problems (A) of unknown or corrupted origin of a sample, (B) losing the identity of a sample and (C) to detect potential contamination of a sample as set out above.

Such information can be used to determine the origin of a single sample, but also the combination of information from different samples that were arranged in a distinct raster can be used. For example, samples were arranged on a 96- or 384-well rack and the information of the positioning on the rack was lost. This can happen if a plate is accidentally turned by 180° without having the plate labelled or other orientation markers on the plate. Moreover, it can occur some of the samples are positioned on wrong positions on the rack e.g. a distinct sample is in position A2 instead of A1 in a 96 well plate. Additionally, systematic shifts e.g. each sample is shifted by one position can be detected. Usually, such errors can result in loosing the identity of single or all samples on the rack and wrong results. The integrity of single or multiple samples, however, can be determined if sample characteristics of the samples are available. Calculating the similarity between the newly acquired mass spectrometer data of one or more samples on the rack and/or well and comparing it with a predetermined sample characteristic of one or more samples on the rack, will allow the identification of errors in the sample positioning and determining the real sample position.

Sample swapping accidentally or purposefully could be avoided. Swapping of a sample is one of the largest problems in various areas where a sample is handled e.g. from biobanking, to large-scale proteomics or other sample analysis to clinical routine. In clinical routine usually a barcode will be applied to track a sample throughout the process. However, barcodes can be swapped or assigned to the wrong person. We propose that a predetermined sample characteristic in a MS-integrity-code that is readable by a mass spectrometry system should be able to track a sample throughout clinical and other analysis. This will allow to match the right sample (and data connected to this sample) to the right person. This solves (at least) the problems (A) of unknown or corrupted origin of a sample and (B) losing the identity of a sample as set out above. Not only will this allow to give the right diagnosis to the right patients but would also prevent purposefully swapping of a sample.

A weighting of distinct factors can be further applied to strengthen the MS-integrity-code. For example, PZP has a high importance as it can distinguish a sample that is originating from a woman or a man. Men have usually low levels of PZP, but some women do also have low levels. In contrast, men almost never have high PZP levels. It would make sense to apply a weighting in the algorithm that PZP and similar behaving proteins are scored differently in different scenarios. The same can be applied for alleles due to the missing value problem of mass spectrometry methods. Identified alleles should result in a higher score than the absence of an allele that can be incorrect due to the missing value problem. Such weightings can be applied to enhance the data integrity code.

Blood plasma can be seen as the most difficult human sample for the analysis of the sample integrity by a mass spectrometry system as plasma has the highest concentration range of all human samples. Therefore, it typically results in the lowest number of proteins that one can detect in a human sample. As a comparison the average number of identified proteins is around 400 protein groups for plasma. The numbers of identified proteins will be much higher in other human samples e.g. in CSF results in over 1,000 proteins and in tissues such as muscle or brain, it can increase over 10,000 proteins (26-28). Therefore, it will be even easier to use such samples to check the integrity of the sample in terms of identifying the origin of a sample.

### Example 2 - Contamination of multiple samples

Next to losing the identity of a sample or swapping samples, the integrity of a sample might get compromised by contamination with one or more other samples. This is problematic as such contamination are likely to falsify the information that can be obtained from this sample. A disease might not be detected if a sample is contaminated by another sample or a disease is diagnosed in a healthy person. Contamination might occur non-purposefully e.g. accidentally if samples of the same individual should be pooled and if instead samples of different individuals are pooled.

Mixing of samples can be detected by an integrity check with a mass spectrometry system, if a MS-integrity-code with a predetermined sample characteristic is available. For this purpose, we mixed a sample of an individual for which a MS-integrity-code has been established with another sample at ratios of 1:1 and 1:10 and mixed the sample with a pool of samples from different individuals (Examples 8, 9, 10; Figure 2 and 3). Figure 8 shows the comparison of quantitative protein levels that are used as predetermined sample characteristics (y-axis) and newly acquired integrity data by a mass spectrometry system (x-axis). A sample of the individual 1 (Ind1_TP1) has been analysed before by MS-based proteomics to establish the MS-integrity-code based on predetermined sample characteristics, which were in this case quantitative protein levels (Figure 8A). Another sample of the same individual has been measured (Ind1_TP2). The comparison of the quantitative protein levels show a high similarity of the two samples (Figure 8A). When samples were contaminated 1:1 with a sample of another individual, this resulted in much lower similarity indicating problems with the sample integrity (Figure 8B). Moreover, we contaminated a sample with another sample in a 1:10 ratio, further lowering the similarity of the quantitative proteome (Figure 8C). The Pearson correlation coefficient decreased after mixing a sample with another sample to 0.96, whereas the uncontaminated samples from the same individual taken at different time points would yield higher Pearson correlation coefficients such as 0.99 (Examples 8 and 2; Figure 2 and 3). Other comparison methods are also possible. Moreover, tracking distinct proteins such as the above-mentioned hormone-regulated proteins such as PZP and SHBG or highly individual-specific proteins such as the apolipoprotein(a) can be applied for detecting corruption of the sample integrity due to contamination with one or more other samples.

Next to protein levels, especially MS-identified alleles are a highly valuable input as a predetermined sample characteristic in the MS-integrity-code for detecting cross-contaminations of a sample. The number of overlapping alleles between the different samples clearly delivered further evidences that a sample was contaminated (Example 8, 9; Figure 2). The alleles are detected as peptide variants and therefore their information is present in a quantitative form, which further allows the determination of the degree of contamination.

We further calculated scores for the identification and the complete MS-integrity-score. Both highlighted that the integrity of the samples had been obscured (Example 8, 9; Figure 3). Additionally, we added a pool of ten individuals to the sample. In the case of the pooled sample, it was obvious due to the high PZP levels that plasma from women was part of the pool (Example 10; Figure 2).

### Examples 3 - Problems in sample processing.

The integrity of a sample can further be influenced by sample processing. Frequent errors in processing of a blood plasma sample are partial coagulation, if an EDTA-container is not shaken after blood taking, erythrocyte lyses occurs due to delays in centrifugation or re-contamination of plasma with platelets. Moreover, the sample quality might be influenced by other factors such as storage or contamination with keratins during processing. Keratins can fall into a sample due to contamination by humans that handle the sample. We propose that it is possible to integrate information of the quality of a sample also in the analysis of the sample integrity by a mass spectrometry system. The falsification of the proteome by contamination due to sample processing will obscure the integrity of a biological sample and should be detectable. Additionally, it is possible to confirm the integrity with regards to the identity of the sample or its potential contaminations (Figure 3). This will also allow to pinpoint the potential issue such as that the sample is contaminated or originating of a different origin than the MS-integrity-code was generated of. As the predetermined sample characteristic, the typical quantitative level of preestablished contamination markers can be used in the MS-integrity-code (Figure 1).

To illustrate the application of a contamination marker as a predetermined sample characteristic in the MS-integrity-code, we prepared a plasma sample with and without sample processing issues for the same person. The integrity with regards to the identity of the sample was given (Example 12; Figure 2 and 3). However, the integrity was obscured as visible in a very low contamination score (2.35E-05) and in a particular low complete MS-integrity-score of 1.56E-05 (Example 12; Figure 3). In a direct comparison of quantitative protein levels, the contamination marker for erythrocyte lysis such as HBA1, HBB, HBD and the contamination marker for platelet contamination such as THBS1, PPBP, PF4 were strongly increased in the sample with processing issues (Figure 9, Ind2_TP2; Example 12; Figure 3). The example 11 illustrates one of the "worst-case scenarios". The new sample in the process was contaminated and from a different individual than the MS-integrity-code sample (Example 11; Figure 2 and 3).

Other possibilities to detect sample contamination are the contamination of a tissue sample with blood or other biofluids. The amount of plasma proteins or proteins from typical blood components such as erythrocytes or platelets can be assessed in a tissue sample. Examples for potential contaminations are the contamination of a biopsy e.g. tumor biopsy or muscle biopsy with blood. Other possibilities are for example the contamination of a tumor sample with a high amount of stroma. Another possibility is the contamination of CSF with blood during sampling. Moreover, issues in the further processing of a sample can also be detected. For example, if a urine sample has been centrifuged or not, will result in different amounts of cells that are present in a sample and this can be detected by a predetermined sample characteristic and a mass spectrometry system for evaluation of the sample integrity. The same is true for biopsies that are not correctly treated after paraffinization to generate formalin-fixed and paraffin embedded tissue.

By comparing the levels of all analysed biomolecules with predetermined sample characteristics using machine learning or other classification methods, the mass spectrometry system for integrity analysis can determine if the analysed sample is different from a previously analysed sample and if a problem during sample processing has occurred.

In the same line, MS can retrieve information on the sample preparation procedure in which proteins are prepared for the MS-measurement (consisting of denaturation, alkylation, digestion, purification). This information can be further integrated to check the sample integrity. This will include missed cleavage rates, alkylation rate, oxidation rate, contamination of lipids and metabolites and many more. These parameters can be integrated as a predetermined sample characteristic in the MS-integrity-code and the mass spectrometry system can be used to confirm the integrity of the sample with regards to the sample processing. This procedure can also be applied to assess whether a distinct enrichment or depletion step was successful or not. It can also detect if such an enrichment step resulted in enrichment of contaminating proteins e.g. platelet or erythrocyte proteins in plasma. For example, enrichment of exosomes from biofluids can be checked for their integrity. Typically, contaminations of plasma exosome fractions by platelets can happen, which would be detected by a MS-integrity-code established for the exosomes.

### Example 4 - Cryptography

The MS-integrity-code can be applied for providing encrypted sample handling of a biological sample from mass spectrometry data. In a laboratory or clinical environment, adequate sample handling in terms of privacy is of utmost importance. This typically is realized by storing experimental data in an encrypted form and limiting access to authorized personnel. Here, several technical approaches are possible. They typically involve creating a key at start and using this for subsequent encryption purposes. Additional layers of security are possible such as multi-factor-authentication. Data privacy is especially critical when dealing with mass spectrometry and other omics data. In contrast to targeted tests that aim to validate the existence of a specific marker or not, a mass spectrometry analysis typically yields information about a plethora of proteins and can provide potentially unwanted information (e.g. detecting markers of a rare disease when making an unrelated check). The predetermined sample characteristics that are combined in the MS-integrity-code can act as a key to encrypt patients' data. The description is only possible with data generated by a mass spectrometry system. The description can be allowed if a distinct similarity distance is achieved or if the similarity is the same. The first might be necessary as for example protein levels changes slightly over time and the second could be used for non-changing data such as alleles or genetic determined protein levels such as apolipoprotein(a) levels. This allows creating a secure sample analysis platform, where only properly acquired sample data can be used to correctly decrypt a code based on predetermined sample characteristics in the MS-integrity-code. Moreover, such a setting might be directly applied not only for having a secured sample analysis platform, but also as a key that gives access to health relevant information of a patients. Such a system may be also used apart from the healthcare sector to encrypt and decrypt data. The MS-integrity-code could be used as a person-bound encryption and decryption system that has a high security level as only the individual that is the origin of the sample can decrypt the information which are encrypted by the predetermined sample characteristics in the MS-integrity-code. As the integrity of the sample has to be ensured e.g. no wrong sample collection can happen, the correct mass spectrometry data key might only be generated under standardized conditions e.g. the blood sample has to be centrifuged at a distinct standardized g-force and only a distinct layer of the plasma column can be used as other layers might have to high platelet contamination.

Assume, a MS-integrity-code is developed at location 1 for a patient. This MS-integrity-code can be used as a key to encrypt patient data. The data can now be shared on public repositories. Sometime later, the patient visits location 2 and performs a mass spectrometry analysis. A sample-specific key will be generated and can be used to decrypt the data shared on public repositories.

Advantageously, the sample key is stored within the patient itself; no secure key storage location is required that can be hacked. Moreover, deciphering of the key will only work if the data is recorded with sufficient reproducibility. If a mass spectrometry system generated a key at location 2, it does not unlock the MS-integrity-code of the public storage, the patient has direct feedback on the quality of the sample. If significant changes between the samples happen, key decryption will be impacted, and the patient could be prevented from accessing previous recordings. This, however, can be advantageous as it prevents combining and interpreting analysis that are fundamentally not comparable or should not be compared. Such cases include if a sample was swapped in the process, contaminated or if the sample integrity was influenced in another way. Moreover, database consistency is forced: Only matching data can be added. This also avoids a common problem when making healthcare data publicly available: Past health conditions could negatively affect healthcare scores (e.g., for healthcare providers). If past data cannot be unlocked, old data records are nullified; only the latest analysis will count. This also allows using emerging tamper-proof technologies, such as blockchain technology, where a past health entry would be stored for eternity. When using blockchain technology, the accessing of data could be stored as well and could provide a layer of security when e.g., stealing a blood sample to achieve a sample key. All data access would be transparently recorded within the public blockchain. There may be an additional layer for the encryption. For example, if mass spectrometry were used to generate the predetermined sample characteristic for the MS-integrity-code, system specific parameters could be added. This can include distinct parameters from the mass spectrometer instrument e.g. distinct types of scan modes, added molecules to the sample that is analyzed or adding distinct chemical modifications to the peptides or proteins during the sample preparation unit of the mass spectrometry system. Such parameters in the MS-integrity-code would allow to generate a pipeline- and/or laboratory-specific key and would render stealing a blood sample and analyzing it to achieve a sample key useless. To add another layer of security the combination of mass spectrometry data with other data types such as sequencing data or immunoassay data could be used for encryption and/or decryption.

### Numbered paragraphs:

1. A mass spectrometry system, the mass spectrometry system comprising:
   a mass spectrometry apparatus configured to provide mass spectrometry data of a biological sample; and
   a computer system configured to:
      process the mass spectrometry data of a biological sample to determine a determined sample characteristic of the biological sample as integrity data of the biological sample; and
      output the integrity data of the biological sample.
2. A computerized method for providing an indication of integrity of a biological sample from mass spectrometry data, the computerized method comprising:
   processing mass spectrometry data of a biological sample to determine a determined sample characteristic of the biological sample as integrity data of the biological sample; and
   outputting the integrity data of the biological sample.
3. The mass spectrometry system according to claim 1 or the computerized method according to claim 2, wherein the integrity data comprises the determined sample characteristic and/or an indication of similarity of the determined sample characteristic of the biological sample and a predetermined sample characteristic.
4. The mass spectrometry system according to any preceding numbered paragraph, wherein the predetermined sample characteristic is an ion with specific characteristics selected from one or more of mass, charge, mass-to-charge-ratio, MS2 fragment spectra, fragment spectra, ion mobility, intensity information, retention time, sequence information and/or unidentified ions.
5. The mass spectrometry system according to any preceding numbered paragraph, wherein the predetermined sample characteristic is a protein or a peptide, preferably a quantified protein or a peptide.
6. The mass spectrometry system according to numbered paragraph 5, wherein the protein is selected from one or more of pregnancy zone protein (PZP), sex hormone binding globulin (SHBG), apolipoprotein(a) (LPA), other apolipoproteins (APOA1, APOB, APOA2, APOA4, APOC1, APOC3, APOC4, APOC2, APOD, APOE), immunoglobulin chains, hemoglobin subunits (HBA1, HBB, HBD, HBG1, HBE, HBZ), carbonic anhydrases (CA1, CA2), peroxiredoxins (PRDX2, PRDX6), catalase (CAT), band 3 anion transport protein (SLC4A1), spectrin chains (SPTA1, SPTB), ankyrin-1 (ANK1), glyceraldehyde-3-phosphate dehydrogenase (GAPDH), superoxide dismutase (SOD1), bisphosphoglycerate mutase (BPGM), actins (ACTB, ACTG1, ACTA1, ACTC1), selenium-binding protein 1 (SELENBP1), protein 4.1 (EPB41), L-lactate dehydrogenase B chain (LDHB), filamin-A (FLNA), talin-1 (TLN1), myosin-9 (MYH9), actin (ACTB), vinculin (VCL), alpha-actinin-1 (ACTN1), tropomyosin alpha-4 chain (TPM3), thrombospondin-1 (THBS1), thrombospondin-4 (THBS4), tubulins (TUBB1, TUBB4B,), 14-3-3 protein zeta/delta (YWHAZ), gelsolin (GSN), tubulin alpha-1B chain (TUBA1B), integrins (ITGA2B), coagulation factors (F13A1, F2, F5, F7, F9, F10, F11, F12), profilin-1 (PFN1), transgelin-2 (TAGLN2), fermitin family homolog 3 (FERMT3), RAS-related proteins (RAP1B), pleckstrin (PLECK) platelet basic protein (PPBP), fibrinogen chains (FGA, FGG, FGB), antithrombin-III (SERPINC1), prothrombin (F2), platelet glycoprotein Ib alpha chain (GP1BA), platelet factor 4 (PF4, PF4v1), extracellular matrix protein 1 (ECM1), clusterin (CLU), desmoplakin (DSP), WD repeat-containing protein 1 (WDR1), attractin (ATRN), platelet glycoprotein V (GP5), plasma serin protease inhibitor (SERPINA5), complement C1r subcomponent-like protein (C1RL), mannosyl-oligosaccharide 1,2-alpha-mannosidase IA (MAN1A1), kininogen-1 (KNG1), cholinesterase (BCHE), polymeric immunoglobulin receptor PIGR), keratins (KRT1, KRT10, KRT17, KRT2, KRT28, KRT9), fructose-bisphosphate aldolase (ALDOA, ALDOB), C-reactive protein (CRP), serum amyloid A proteins (SAA1, SAA2, SAA4), pregnancy specific pregnancy-specific beta-1-glycoprotein 1 (PSG1), pregnancy-specific beta-1-glycoprotein 9 (PSG9), actin-related protein 2 (ACTR2), prelaminA/C (LMNA), septin-9 (SEPTN9), peptidyl-prolyl cis-trans isomerase (FKBP2), V-type proton ATPase subunit B, brain isoform (ATP6V1B2).
7. Preferably, the protein is selected from PZP, LPA, APOE, HBA1, FLNA, FGA, KRT9, CRP, PSG1, ACTR2.
8. The mass spectrometry system according to any one of numbered paragraphs 1 to 3, wherein, the predetermined sample characteristic is a quantified peptide.
9. The mass spectrometry system according to any one of numbered paragraphs 1 to 3, wherein the predetermined sample characteristic is a post-translational modification, preferably a quantified post-translational modification.
10. The mass spectrometry system according to numbered paragraph 9, wherein the post-translational modification is a phosphorylation, glycosylation, glycation, ubiquitination, S-nitrosylation, methylation, N-acetylation, SUMOylation, and/or lipidation.
11. The mass spectrometry system according to any of one numbered paragraph 1 to 3, wherein the predetermined sample characteristic is an allele and/or a variant peptide.
12. The mass spectrometry system according to numbered paragraph 11, wherein the allele is selected from one or more of the following genes listed by gene names: LPA, PON1, GC, APOB, APOE, AGT, A1BG, A2M, ABCC2, ACTB, ACTC1, ACTA1, ACTA2, ACTG2, ADIPOQ, AFM, AFP, AHNAK, AHSG, ALB, ALDH1A1, APOA4, APOH, APOL1, C3, HEL-S-62p, C4A, C7, CP, CPN2, F5, FGG, DKFZp779N0926, HBA1, HBB, HBD, HP, LBP, PGLYRP2, SERPINA1, SERPINF1, SERPINF2, F10, F11, F12, F13B, F2, F7, F9, SERPING1, TF, TTR, HEL111, ALDH1A3, ALDOA, ALDOB, AMBP, ANGPTL3, ANPEP, APCS, APEH, APMAP, APOA1, APOA2, APOC1, APOC3, APOC4, APOC4-APOC2, APOC2, APOD, APOF, APOM, ARHGAP1, ARSB, ATP1A4, ATP6V1A, ATRN, ATRNL1, AZGP1, B2M, BCHE, BLK, BLVRB, BTD, C150rf41, C1QA, C1QB, C1QC, C1R, C1RL, C1S, C2, C3, C4B, C4BPA, C4BPB, C5, C6, C8A, C8B, C8G, C9, CA1, CA2, CABIN1, CALD1, CALM1, CALM2, CALM3, CALR, CARD9, CARD11, CAT, CD14, CD163, CD44, CD5L, CDH5, CDHR2, CEP164, CFB, CFD, CFH, CFHR3, CFHR4, CFI, CFL1, CHGA, CHI3L1, CHIT1, CHRNB1, CKM, CLEC3B, CLTC, CLTCL1, CLU, CNDP1, CNTN3, COL18A1, COL6A3, COLEC11, COPE, CPB2, CPN1, CPS1, CRISP3, CRP, CRTAC1, CRYAB, CRYZ, CSH2, CSH1, CST3, CTSA, CTSD, CUBN, DBH, ECM1, EIF4A1, ENO1, ERLIN1, ERN1, ETFA, EXOC1, FABP4, FAH, FAM153A, FAM162A, FBLN1, FCGBP, FCGR3A, FCN2, FCN3, FETUB, FGA, FGB, FGFR2, FGG, FGL1, FITM1, FKBP4, FLII, FLOT2, FN1, GAPDH, GBA, GCA, GDI2, GGH, GLUD1, GLUD2, GP1BA, GPC6, GPLD1,GPRC5C, GPX3, GSN, GSTM4, HABP2, HADH, HARS, HBG2, HEXA, HGFAC, HIST1H4A, HLA-A, HLA-H, HLA-C, HPR, HPX, HRG, HSP90AA1 ,HSP90B1, HSPA5, HSPA8, HSPG2, ICAM1, ICAM2, IGFALS, IGFBP3, IGFBP6, IL1RAP, INTS4, ITIH1, ITIH2, ITIH3, ITIH4, KCTD12, KIAA0319L, KLKB1, KNG1, KPNB1, KRT24, LAMB2, LCAT, LCN2, LCP1, LDHA, LDHB, LGALS3BP, LILRB1, LILRA1, LOC93432, LRG1, LRP2, LTF, LUM, LYVE1, LYZ, MANBA, MARCKS, MASP1, MASP2, MB, MBL2, MEI1, MIA3, MMP9, MMRN1, MPO, MST1, MST1L, MUC4, MYH11, MYH14, MYO1A, MYO1B, MYO1D, NCF4, NCKIPSD, NEO1, NIN, NRP2, ORM1, ORM2, PC, PCCA, PCDHA8, PCOLCE, PCYOX1, PDIA4, PEBP1, PF4V1, PF4, PFN1, PI16, PIGR, PLCD1, PLCG2, PLEC, PLG, PLS1, PLTP, PON3, PPA1, PPBP, PPIA, PPIL1, PRAP1, PRCC, PRDX2, PRG2, PRG4, PROC, PROCR, PROS1, PROZ, PRSS2, PSG1, PSMB1, PSMC6, PSMD2, PTGDS, PTPRF, PUS10, PZP, QSOX1, RAB21, RAN, RANBP2, RBP1, RBP4, RECK, REG1A, RNASE4, RNF111, RPL10, S100A9, SAA1, SAA2, SAA4, SDC1, SELL, SEPP1, SERPINA10, SERPINA3, SERPINA4, SERPINA5, SERPINA6, SERPINA7, SERPINB6, SERPINC1, SERPIND1, SFTPB, SHBG, SLC12A3,SLC3A2, SNCA, SOD3, SPP1, SPTA1, SPTAN1, SPTB, SRGN, STXBP5L, SUMO2, SUMO3, SUMO4, TAGLN2, TCP1, TFRC, TGFBI, THBS1, TIMP1, TMSB10, TMSB4X, TNC, TNXB, TOR3A, TRHDE, TTN, TTR, TXN, UBC, UBB, RPS27A, UBA52, UBBP4, UCHL3, UGT8, VASN, VCAM1, VNN1, VSIG4, VTN, VWF, YWHAE, ZNF256, ZNF652, preferably LPA, PON1, GC, APOB, APOE, AGT, A1BG, A2M, ABCC2, ACTB, ACTC1, ACTA1, ACTA2, ACTG2, ADIPOQ, AFM, AFP, AHNAK, AHSG, ALB, ALDH1A1, APOA4, APOH, APOL1, C3, HEL-S-62p, C4A, C7, CP, CPN2, F5, FGG, DKFZp779N0926, HBA1, HBB, HBD, HP, LBP, PGLYRP2, SERPINA1, SERPINF1, SERPINF2, F10, F11, F12, F13B, F2, F7, F9, SERPING1, TF, TTR, HEL111, ALDH1A3, ALDOA, ALDOB, AMBP, ANGPTL3, ANPEP, APCS, APEH, APMAP, APOA1, APOA2, more preferably LPA, PON1, GC, APOB, APOE, AGT, A1BG, A2M, ADIPOQ, AFM, ALB, APOA4, APOL1, C3, CP, CPN2, F5, FGG, preferably LPA, PON1, GC, APOB, APOE, AGT, A1BG, A2M, ADIPOQ, AFM.
13. The mass spectrometry system according to any of one numbered paragraph 1 to 3, wherein the predetermined sample characteristic comprises biomolecules such as lipids, small molecules such as drugs or metabolites, carbohydrates, preferably quantified biomolecules.
14. The mass spectrometry system according to any of one numbered paragraph 1 to 3, wherein the predetermined sample characteristic comprises anthropometric data, e.g. height, weight, BMI.
15. The mass spectrometry system according to any of one numbered paragraph 1 to 3, wherein the predetermined sample characteristic comprises a medical relevant information such as clinical analysis, e.g. HDL, LDL, cholesterol, C-reactive protein (CRP), hemoglobin (Hb), red blood cell (RBC), white blood cell (WBC) count, lymphocyte count, neutrophil count, platelet count (PLTs), mean platelet volume (MPV), platelet distribution width (PDW), erythrocyte sedimentation rate (ESR), health or disease state, genetic disorder and/or medication.
16. The mass spectrometry system according to any of one numbered paragraph 1 to 3, wherein the predetermined sample characteristic comprises information on sex, pregnancy, ethnicity.
17. The mass spectrometry system according to any of one numbered paragraph 1 to 3, wherein the predetermined sample characteristic is information on metadata about an individual such as genomic and/or transcriptomic data.
18. The mass spectrometry system according to any preceding numbered paragraph, wherein the computer is configured to process the mass spectrometry data to determine a sample characteristic to generate a similarity metrics based on defined metrics between samples analysed by mass spectrometry and/or a predetermined sample characteristic from a sample analysed by mass spectrometry to and/or one or more non-mass spectrometry generated sample characteristics; and output an indication of the integrity of the biological sample.
19. A computerized method for providing an indication of integrity of a biological sample from mass spectrometry data, the computerized method comprising:
   processing mass spectrometry data of a biological sample to determine a determined sample characteristic of the biological sample as integrity data of the biological sample; and outputting the integrity data of the biological sample.
20. The mass spectrometry detection method of numbered paragraph 19, wherein the biological sample is a human biological sample.
21. The mass spectrometry detection method of according to numbered paragraph 19, wherein the biological sample comprises blood, plasma, serum, urine, cerebrospinal fluid, saliva, tears, stool, gastric juice, tissue, fresh tissue, processed tissue, biopsies, liquid biopsies, hair, bone.
22. The mass spectrometry detection method of according to the numbered paragraph 19 to 21, wherein the predetermined sample characteristic is a ion with specific characteristics selected from mass, charge, mass-to-charge-ratio, MS2 fragment information, fragment spectra, ion mobility, intensity information, retention time and/or unidentified ions.
23. The mass spectrometry detection method according to numbered paragraph 19 to 21, wherein the predetermined sample characteristic is a protein or a peptide, preferably a quantified protein or a peptide.
24. The mass spectrometry detection method according to numbered paragraph 23, wherein the protein is selected from one or more of pregnancy zone protein (PZP), sex hormone binding globulin (SHBG), apolipoprotein(a) (LPA), other apolipoproteins (APOA1, APOB, APOA2, APOA4, APOC1, APOC3, APOC4, APOC2, APOD, APOE), immunoglobulin chains, hemoglobin subunits (HBA1, HBB, HBD, HBG1, HBE, HBZ), carbonic anhydrases (CA1, CA2), peroxiredoxins (PRDX2, PRDX6), catalase (CAT), band 3 anion transport protein (SLC4A1), spectrin chains (SPTA1, SPTB), ankyrin-1 (ANK1), glyceraldehyde-3-phosphate dehydrogenase (GAPDH), superoxide dismutase (SOD1), bisphosphoglycerate mutase (BPGM), actins (ACTB, ACTG1, ACTA1, ACTC1), selenium-binding protein 1 (SELENBP1), protein 4.1 (EPB41), L-lactate dehydrogenase B chain (LDHB), filamin-A (FLNA), talin-1 (TLN1), myosin-9 (MYH9), actin (ACTB), vinculin (VCL), alpha-actinin-1 (ACTN1), tropomyosin alpha-4 chain (TPM3), thrombospondin-1 (THBS1), thrombospondin-4 (THBS4), tubulins (TUBB1, TUBB4B,), 14-3-3 protein zeta/delta (YWHAZ), gelsolin (GSN), tubulin alpha-1B chain (TUBA1B), integrins (ITGA2B), coagulation factors (F13A1, F2, F5, F7, F9, F10, F11, F12), profilin-1 (PFN1), transgelin-2 (TAGLN2), fermitin family homolog 3 (FERMT3), RAS-related proteins (RAP1B), pleckstrin (PLECK) platelet basic protein (PPBP), fibrinogen chains (FGA, FGG, FGB), antithrombin-III (SERPINC1), prothrombin (F2), platelet glycoprotein Ib alpha chain (GP1BA), platelet factor 4 (PF4, PF4v1), extracellular matrix protein 1 (ECM1), clusterin (CLU), desmoplakin (DSP), WD repeat-containing protein 1 (WDR1), attractin (ATRN), platelet glycoprotein V (GP5), plasma serin protease inhibitor (SERPINA5), complement C1r subcomponent-like protein (C1RL), mannosyl-oligosaccharide 1,2-alpha-mannosidase IA (MAN1A1), kininogen-1 (KNG1), cholinesterase (BCHE), polymeric immunoglobulin receptor PIGR), keratins (KRT1, KRT10, KRT17, KRT2, KRT28, KRT9), fructose-bisphosphate aldolase (ALDOA, ALDOB), C-reactive protein (CRP), serum amyloid A proteins (SAA1, SAA2, SAA4), pregnancy specific pregnancy-specific beta-1-glycoprotein 1 (PSG1), pregnancy-specific beta-1-glycoprotein 9 (PSG9), actin-related protein 2 (ACTR2), prelaminA/C (LMNA), septin-9 (SEPTN9), peptidyl-prolyl cis-trans isomerase (FKBP2), V-type proton ATPase subunit B, brain isoform (ATP6V1B2), preferably, the protein is selected from PZP, LPA, APOE, HBA1, FLNA, FGA, KRT9, CRP, PSG1, ACTR2.
25. The mass spectrometry detection method of according to any of one numbered paragraph 19 to 21, wherein, the predetermined sample characteristic is a quantified peptide.
26. The mass spectrometry detection method of according to any of one numbered paragraph 19 to 21, wherein the predetermined sample characteristic is a post-translational modification, preferably a quantified post-translational modification.
27. The mass spectrometry detection method of according to numbered paragraph 26, wherein the post-translational modification is a phosphorylation, glycosylation, glycation, ubiquitination, S-nitrosylation, methylation, N-acetylation, SUMOylation, and/or lipidation.
28. The mass spectrometry detection method of according to any of one numbered paragraph 19 to 21, wherein the predetermined sample characteristic is an allele and/or variant peptide.
29. The mass spectrometry detection method of according to numbered paragraph 28, wherein the allele is selected from one or more of the following genes listed by gene names: LPA, PON1, GC, APOB, APOE, AGT, A1BG, A2M, ABCC2, ACTB, ACTC1, ACTA1, ACTA2, ACTG2, ADIPOQ, AFM, AFP, AHNAK, AHSG, ALB, ALDH1A1, APOA4, APOH, APOL1, C3, HEL-S-62p, C4A, C7, CP, CPN2, F5, FGG, DKFZp779N0926, HBA1, HBB, HBD, HP, LBP, PGLYRP2, SERPINA1, SERPINF1, SERPINF2, F10, F11, F12, F13B, F2, F7, F9, SERPING1, TF, TTR, HEL111, ALDH1A3, ALDOA, ALDOB, AMBP, ANGPTL3, ANPEP, APCS, APEH, APMAP, APOA1, APOA2, APOC1, APOC3, APOC4, APOC4-APOC2, APOC2, APOD, APOF, APOM, ARHGAP1, ARSB, ATP1A4, ATP6V1A, ATRN, ATRNL1, AZGP1, B2M, BCHE, BLK, BLVRB, BTD, C150rf41, C1QA, C1QB, C1QC, C1R, C1RL, C1S, C2, C3, C4B, C4BPA, C4BPB, C5, C6, C8A, C8B, C8G, C9, CA1, CA2, CABIN1, CALD1, CALM1, CALM2, CALM3, CALR, CARD9, CARD11, CAT, CD14, CD163, CD44, CD5L, CDH5, CDHR2, CEP164, CFB, CFD, CFH, CFHR3, CFHR4, CFI, CFL1, CHGA, CHI3L1, CHIT1, CHRNB1, CKM, CLEC3B, CLTC, CLTCL1, CLU, CNDP1, CNTN3, COL18A1, COL6A3, COLEC11, COPE, CPB2, CPN1, CPS1, CRISP3, CRP, CRTAC1, CRYAB, CRYZ, CSH2, CSH1, CST3, CTSA, CTSD, CUBN, DBH, ECM1, EIF4A1, ENO1, ERLIN1, ERN1, ETFA, EXOC1, FABP4, FAH, FAM153A, FAM162A, FBLN1, FCGBP, FCGR3A, FCN2, FCN3, FETUB, FGA, FGB, FGFR2, FGG, FGL1, FITM1, FKBP4, FLII, FLOT2, FN1, GAPDH, GBA, GCA, GDI2, GGH, GLUD1, GLUD2, GP1BA, GPC6, GPLD1,GPRC5C, GPX3, GSN, GSTM4, HABP2, HADH, HARS, HBG2, HEXA, HGFAC, HIST1H4A, HLA-A, HLA-H, HLA-C, HPR, HPX, HRG, HSP90AA1 ,HSP90B1, HSPA5, HSPA8, HSPG2, ICAM1, ICAM2, IGFALS, IGFBP3, IGFBP6, IL1RAP, INTS4, ITIH1, ITIH2, ITIH3, ITIH4, KCTD12, KIAA0319L, KLKB1, KNG1, KPNB1, KRT24, LAMB2, LCAT, LCN2, LCP1, LDHA, LDHB, LGALS3BP, LILRB1, LILRA1, LOC93432, LRG1, LRP2, LTF, LUM, LYVE1, LYZ, MANBA, MARCKS, MASP1, MASP2, MB, MBL2, MEI1, MIA3, MMP9, MMRN1, MPO, MST1, MST1L, MUC4, MYH11, MYH14, MYO1A, MYO1B, MYO1D, NCF4, NCKIPSD, NEO1, NIN, NRP2, ORM1, ORM2, PC, PCCA, PCDHA8, PCOLCE, PCYOX1, PDIA4, PEBP1, PF4V1, PF4, PFN1, PI16, PIGR, PLCD1, PLCG2, PLEC, PLG, PLS1, PLTP, PON3, PPA1, PPBP, PPIA, PPIL1, PRAP1, PRCC, PRDX2, PRG2, PRG4, PROC, PROCR, PROS1, PROZ, PRSS2, PSG1, PSMB1, PSMC6, PSMD2, PTGDS, PTPRF, PUS10, PZP, QSOX1, RAB21, RAN, RANBP2, RBP1, RBP4, RECK, REG1A, RNASE4, RNF111, RPL10, S100A9, SAA1, SAA2, SAA4, SDC1, SELL, SEPP1, SERPINA10, SERPINA3, SERPINA4, SERPINA5, SERPINA6, SERPINA7, SERPINB6, SERPINC1, SERPIND1, SFTPB, SHBG, SLC12A3,SLC3A2, SNCA, SOD3, SPP1, SPTA1, SPTAN1, SPTB, SRGN, STXBP5L, SUMO2, SUMO3, SUMO4, TAGLN2, TCP1, TFRC, TGFBI, THBS1, TIMP1, TMSB10, TMSB4X, TNC, TNXB, TOR3A, TRHDE, TTN, TTR, TXN, UBC, UBB, RPS27A, UBA52, UBBP4, UCHL3, UGT8, VASN, VCAM1, VNN1, VSIG4, VTN, VWF, YWHAE, ZNF256, ZNF652, preferably LPA, PON1, GC, APOB, APOE, AGT, A1BG, A2M, ABCC2, ACTB, ACTC1, ACTA1, ACTA2, ACTG2, ADIPOQ, AFM, AFP, AHNAK, AHSG, ALB, ALDH1A1, APOA4, APOH, APOL1, C3, HEL-S-62p, C4A, C7, CP, CPN2, F5, FGG, DKFZp779N0926, HBA1, HBB, HBD, HP, LBP, PGLYRP2, SERPINA1, SERPINF1, SERPINF2, F10, F11, F12, F13B, F2, F7, F9, SERPING1, TF, TTR, HEL111, ALDH1A3, ALDOA, ALDOB, AMBP, ANGPTL3, ANPEP, APCS, APEH, APMAP, APOA1, APOA2, more preferably LPA, PON1, GC, APOB, APOE, AGT, A1 BG, A2M, ADIPOQ, AFM, ALB, APOA4, APOL1, C3, CP, CPN2, F5, FGG, preferably LPA, PON1, GC, APOB, APOE, AGT, A1BG, A2M, ADIPOQ, AFM.
30. The mass spectrometry detection method of according to any of one numbered paragraph 19 to 21, wherein the predetermined sample characteristic comprises biomolecules such as lipids, small molecules such as drugs or metabolites, carbohydrates, preferably quantified biomolecules.
31. The mass spectrometry detection method of according to any of one numbered paragraph 19 to 21, wherein the predetermined sample characteristic is an ion that is not specifically identified, or an unidentified ion or raw data.
32. The mass spectrometry detection method of according to any of one numbered paragraph 19 to 21, wherein the predetermined sample characteristic comprises anthropometric data, e.g. height, weight, BMI.
33. The mass spectrometry detection method of according to any of one numbered paragraph 19 to 21, wherein the predetermined sample characteristic comprises a medical relevant information such as clinical analysis, e.g. HDL, LDL, cholesterol, C-reactive protein (CRP), hemoglobin (Hb), red blood cell (RBC), white blood cell (WBC) count, lymphocyte count, neutrophil count, platelet count (PLTs), mean platelet volume (MPV), platelet distribution width (PDW), erythrocyte sedimentation rate (ESR), health or disease state, genetic disorder and/or medication.
34. The mass spectrometry detection method of according to any of one numbered paragraph 19 to 21, wherein the predetermined sample characteristic comprises information on sex, pregnancy, ethnicity.
35. The mass spectrometry detection method of according to any of one numbered paragraph 19 to 21, wherein the predetermined sample characteristic is information on metadata about an individual.
36. The mass spectrometry system according to any preceding numbered paragraph, wherein the computer configured to process the mass spectrometry determined sample characteristic to generate a similarity, e.g. based on defined metrics, between samples analysed by mass spectrometry or sample analysed by mass spectrometry to another non-mass spectrometry predetermined sample characteristic.
37. A computer program for controlling the method of numbered paragraph 19 to 36.
38. A computer-readable medium comprising instructions for controlling the method of numbered paragraph 19 to 37.
39. A computer system for processing mass spectrometry data, the computer system is configured to process the mass spectrometry data to determine a sample characteristic to generate a similarity metrics based on defined metrics between samples analysed by mass spectrometry and/or a predetermined sample characteristic from a sample analysed by mass spectrometry to and/or one or more non-mass spectrometry generated sample characteristics; and output an indication of the integrity of the biological sample.
40. Use of mass spectrometry data to assess the integrity of a biological sample.
41. The mass spectrometry detection method of according to any of one of the numbered paragraphs 19 to 21, wherein the predetermined sample characteristic is a combination of the previously mentioned sample characteristics.
42. A sample characteristic combination being determined by mathematical optimization (e.g. machine learning).
43. The predetermined sample characteristic integrated in a MS-integrity-code and a mass spectrometry system to provide mass spectrometer data can be applied in cryptography. A MS-integrity-code can be generated to encrypt data e.g. data in a healthcare system. Only having a mass spectrometry system to provide mass spectrometry data can confirm the integrity of the sample and in this way act as a key to decrypt the stored healthcare data. The MS-integrity-code can act as a key from the sample itself. This allows creating a secure platform, where only correctly acquired sample data can be used to correctly decrypt the sample-specific code.
44. A machine-readable code such as barcode or QR code encoding a predetermined sample characteristic of a MS-integrity-code that can be compared with newly acquired mass spectrometer data of a biological sample.
45. Use of a predetermined sample characteristic and a mass spectrometry system configured to provide mass spectrometer data, wherein the use is in cryptography.
46. The use according to numbered paragraph 45, comprising a predetermined sample characteristic for encrypting data to provide encrypted data and a mass spectrometry system configured to provide mass spectrometer data for decrypting the encrypted data.
47. The use according to the numbered paragraphs 44 to 46, wherein a chemical reaction is used to facilitate chemical modifications on biomolecules to add a specific signature to the MS-integrity-code.

## Claims

1. A mass spectrometry system, the mass spectrometry system comprising:
a mass spectrometry apparatus configured to provide mass spectrometry data of a biological sample; and
a computer system configured to:
process the mass spectrometry data of a biological sample to determine a determined sample characteristic of the biological sample as integrity data of the biological sample; and
output the integrity data of the biological sample.

2. A computerized method for providing an indication of integrity of a biological sample from mass spectrometry data, the computerized method comprising:
processing mass spectrometry data of a biological sample to determine a determined sample characteristic of the biological sample as integrity data of the biological sample; and
outputting the integrity data of the biological sample.

3. The mass spectrometry system according to claim 1 or the computerized method according to claim 2, wherein the integrity data comprises the determined sample characteristic and/or an indication of similarity of the determined sample characteristic of the biological sample and a predetermined sample characteristic.

4. The mass spectrometry system according to claim 1 or the computerized method according to claim 2, wherein the biological sample is a human biological sample, and/or wherein the biological sample comprises one or more of blood, plasma, serum, urine, cerebrospinal fluid, saliva, tears, stool, gastric juice, tissue, fresh tissue, fixed tissue, e.g. formalin-fixed paraffin-embedded tissue, processed tissue, biopsies, liquid biopsies, hair, and/or bone.

5. The mass spectrometry system or the computerized method according to any preceding claim, wherein the predetermined sample characteristic is an ion with specific characteristics selected from one or more of mass, charge, mass-to-charge-ratio, fragment spectra, MS2 fragment spectra, ion mobility, intensity information and or retention time.

6. The mass spectrometry system or the computerized method according to any one of claims 1 to 4, wherein predetermined sample characteristic is a protein or a peptide, preferably a quantified protein or a quantified peptide.

7. The mass spectrometry system or the computerized method according to claim 6, wherein the protein is selected from one or more of pregnancy zone protein (PZP), sex hormone binding globulin (SHBG), apolipoprotein(a) (LPA), other apolipoproteins (APOA1, APOB, APOA2, APOA4, APOC1, APOC3, APOC4, APOC2, APOD, APOE), immunoglobulin chains, hemoglobin subunits (HBA1, HBB, HBD, HBG1, HBE, HBZ), carbonic anhydrases (CA1, CA2), peroxiredoxins (PRDX2, PRDX6), catalase (CAT), band 3 anion transport protein (SLC4A1), spectrin chains (SPTA1, SPTB), ankyrin-1 (ANK1), glyceraldehyde-3-phosphate dehydrogenase (GAPDH), superoxide dismutase (SOD1), bisphosphoglycerate mutase (BPGM), actins (ACTB, ACTG1, ACTA1, ACTC1), selenium-binding protein 1 (SELENBP1), protein 4.1 (EPB41), L-lactate dehydrogenase B chain (LDHB), filamin-A (FLNA), talin-1 (TLN1), myosin-9 (MYH9), actin (ACTB), vinculin (VCL), alpha-actinin-1 (ACTN1), tropomyosin alpha-4 chain (TPM3), thrombospondin-1 (THBS1), thrombospondin-4 (THBS4), tubulins (TUBB1, TUBB4B,), 14-3-3 protein zeta/delta (YWHAZ), gelsolin (GSN), tubulin alpha-1B chain (TUBA1B), integrins (ITGA2B), coagulation factors (F13A1, F2, F5, F7, F9, F10, F11, F12), profilin-1 (PFN1), transgelin-2 (TAGLN2), fermitin family homolog 3 (FERMT3), RAS-related proteins (RAP1B), pleckstrin (PLECK) platelet basic protein (PPBP), fibrinogen chains (FGA, FGG, FGB), antithrombin-III (SERPINC1), prothrombin (F2), platelet glycoprotein Ib alpha chain (GP1BA), platelet factor 4 (PF4, PF4v1), extracellular matrix protein 1 (ECM1), clusterin (CLU), desmoplakin (DSP), WD repeat-containing protein 1 (WDR1), attractin (ATRN), platelet glycoprotein V (GP5), plasma serin protease inhibitor (SERPINA5), complement C1rsubcomponent-like protein (C1RL), mannosyl-oligosaccharide 1,2-alpha-mannosidase IA (MAN1A1), kininogen-1 (KNG1), cholinesterase (BCHE), polymeric immunoglobulin receptor PIGR), keratins (KRT1, KRT10, KRT17, KRT2, KRT28, KRT9), fructose-bisphosphate aldolase (ALDOA, ALDOB), C-reactive protein (CRP), serum amyloid A proteins (SAA1, SAA2, SAA4), pregnancy specific pregnancy-specific beta-1-glycoprotein 1 (PSG1), pregnancy-specific beta-1-glycoprotein 9 (PSG9), actin-related protein 2 (ACTR2), prelaminA/C (LMNA), septin-9 (SEPTN9), peptidyl-prolyl cis-trans isomerase (FKBP2), V-type proton ATPase subunit B, brain isoform (ATP6V1B2), preferably the protein is selected from PZP, LPA, APOE, HBA1, FLNA, FGA, KRT9, CRP, PSG1, ACTR2.

8. The mass spectrometry system or the computerized method according to any of one of the preceding claims, wherein the predetermined sample characteristic is a post-translational modification, preferably a quantified post-translational modification, preferably wherein the post-translational modification is a phosphorylation, glycosylation, glycation, ubiquitination, S-nitrosylation, methylation, N-acetylation, SUMOylation, and/or lipidation.

9. The mass spectrometry system or the computerized method according to any of one of the preceding claims, wherein the predetermined sample characteristic is an allele and/or a variant peptide.

10. The mass spectrometry system or the computerized method according to claim 9, wherein the allele is selected from one or more of the following genes listed by gene names: LPA, PON1, GC, APOB, APOE, AGT, A1BG, A2M, ABCC2, ACTB, ACTC1, ACTA1, ACTA2, ACTG2, ADIPOQ, AFM, AFP, AHNAK, AHSG, ALB, ALDH1A1, APOA4, APOH, APOL1, C3, HEL-S-62p, C4A, C7, CP, CPN2, F5, FGG, DKFZp779N0926, HBA1, HBB, HBD, HP, LBP, PGLYRP2, SERPINA1, SERPINF1, SERPINF2, F10, F11, F12, F13B, F2, F7, F9, SERPING1, TF, TTR, HEL111, ALDH1A3, ALDOA, ALDOB, AMBP, ANGPTL3, ANPEP, APCS, APEH, APMAP, APOA1, APOA2, APOC1, APOC3, APOC4, APOC4-APOC2, APOC2, APOD, APOF, APOM, ARHGAP1, ARSB, ATP1A4, ATP6V1A, ATRN, ATRNL1, AZGP1, B2M, BCHE, BLK, BLVRB, BTD, C150rf41, C1QA, C1QB, C1QC, C1R, C1RL, C1S, C2, C3, C4B, C4BPA, C4BPB, C5, C6, C8A, C8B, C8G, C9, CA1, CA2, CABIN1, CALD1, CALM1, CALM2, CALM3, CALR, CARD9, CARD11, CAT, CD14, CD163, CD44, CD5L, CDH5, CDHR2, CEP164, CFB, CFD, CFH, CFHR3, CFHR4, CFI, CFL1, CHGA, CHI3L1, CHIT1, CHRNB1, CKM, CLEC3B, CLTC, CLTCL1, CLU, CNDP1, CNTN3, COL18A1, COL6A3, COLEC11, COPE, CPB2, CPN1, CPS1, CRISP3, CRP, CRTAC1, CRYAB, CRYZ, CSH2, CSH1, CST3, CTSA, CTSD, CUBN, DBH, ECM1, EIF4A1, ENO1, ERLIN1, ERN1, ETFA, EXOC1, FABP4, FAH, FAM153A, FAM162A, FBLN1, FCGBP, FCGR3A, FCN2, FCN3, FETUB, FGA, FGB, FGFR2, FGG, FGL1, FITM1, FKBP4, FLII, FLOT2, FN1, GAPDH, GBA, GCA, GDI2, GGH, GLUD1, GLUD2, GP1BA, GPC6, GPLD1,GPRC5C, GPX3, GSN, GSTM4, HABP2, HADH, HARS, HBG2, HEXA, HGFAC, HIST1H4A, HLA-A, HLA-H, HLA-C, HPR, HPX, HRG, HSP90AA1 ,HSP90B1, HSPA5, HSPA8, HSPG2, ICAM1, ICAM2, IGFALS, IGFBP3, IGFBP6, IL1RAP, INTS4, ITIH1, ITIH2, ITIH3, ITIH4, KCTD12, KIAA0319L, KLKB1, KNG1, KPNB1, KRT24, LAMB2, LCAT, LCN2, LCP1, LDHA, LDHB, LGALS3BP, LILRB1, LILRA1, LOC93432, LRG1, LRP2, LTF, LUM, LYVE1, LYZ, MANBA, MARCKS, MASP1, MASP2, MB, MBL2, MEI1, MIA3, MMP9, MMRN1, MPO, MST1, MST1L, MUC4, MYH11, MYH14, MYO1A, MYO1B, MYO1D, NCF4, NCKIPSD, NEO1, NIN, NRP2, ORM1, ORM2, PC, PCCA, PCDHA8, PCOLCE, PCYOX1, PDIA4, PEBP1, PF4V1, PF4, PFN1, PI16, PIGR, PLCD1, PLCG2, PLEC, PLG, PLS1, PLTP, PON3, PPA1, PPBP, PPIA, PPIL1, PRAP1, PRCC, PRDX2, PRG2, PRG4, PROC, PROCR, PROS1, PROZ, PRSS2, PSG1, PSMB1, PSMC6, PSMD2, PTGDS, PTPRF, PUS10, PZP, QSOX1, RAB21, RAN, RANBP2, RBP1, RBP4, RECK, REG1A, RNASE4, RNF111, RPL10, S100A9, SAA1, SAA2, SAA4, SDC1, SELL, SEPP1, SERPINA10, SERPINA3, SERPINA4, SERPINA5, SERPINA6, SERPINA7, SERPINB6, SERPINC1, SERPIND1, SFTPB, SHBG, SLC12A3,SLC3A2, SNCA, SOD3, SPP1, SPTA1, SPTAN1, SPTB, SRGN, STXBP5L, SUMO2, SUMO3, SUMO4, TAGLN2, TCP1, TFRC, TGFBI, THBS1, TIMP1, TMSB10, TMSB4X, TNC, TNXB, TOR3A, TRHDE, TTN, TTR, TXN, UBC, UBB, RPS27A, UBA52, UBBP4, UCHL3, UGT8, VASN, VCAM1, VNN1, VSIG4, VTN, VWF, YWHAE, ZNF256, ZNF652, preferably LPA, PON1, GC, APOB, APOE, AGT, A1BG, A2M, ABCC2, ACTB, ACTC1,ACTA1, ACTA2,ACTG2, ADIPOQ, AFM, AFP, AHNAK, AHSG, ALB, ALDH1A1, APOA4, APOH, APOL1, C3,HEL-S-62p, C4A, C7, CP, CPN2, F5, FGG, DKFZp779N0926, HBA1, HBB, HBD, HP, LBP, PGLYRP2, SERPINA1, SERPINF1, SERPINF2, F10, F11, F12, F13B, F2, F7, F9, SERPING1, TF, TTR,HEL111, ALDH1A3, ALDOA, ALDOB, AMBP, ANGPTL3, ANPEP, APCS, APEH, APMAP, APOA1, APOA2, more preferably LPA, PON1, GC, APOB, APOE, AGT, A1BG, A2M, ADIPOQ, AFM, ALB, APOA4, APOL1, C3, CP, CPN2, F5, FGG, preferably LPA, PON1, GC, APOB, APOE, AGT, A1BG, A2M, ADIPOQ, AFM.

11. The mass spectrometry system or the computerized method according to any preceding claim, wherein the predetermined sample characteristic comprises biomolecules such as lipids, small molecules such as drugs or metabolites, carbohydrates, preferably quantified biomolecules.

12. The mass spectrometry system or the computerized method according to any preceding claim, wherein at least one of the predetermined sample characteristics comprises anthropometric data, e.g. height, weight, BMI, and/or information on sex, pregnancy, ethnicity and/or wherein the predetermined sample characteristic comprises medical relevant information such as clinical analysis, e.g. HDL, LDL, cholesterol, C-reactive protein (CRP), hemoglobin (Hb), red blood cell (RBC), white blood cell (WBC) count, lymphocyte count, neutrophil count, platelet count (PLTs), mean platelet volume (MPV), platelet distribution width (PDW), erythrocyte sedimentation rate (ESR), health or disease state, genetic disorder and/or medication.

13. The mass spectrometry system or the computerized method according to any preceding claim, wherein the computer is configured to process the mass spectrometry data to determine a sample characteristic to generate a similarity metric between samples analysed by mass spectrometry and a predetermined sample characteristic from a sample analysed by mass spectrometry and/or other non-mass spectrometry generated a sample characteristic; and output an indication of the integrity of the biological sample.

14. A machine-readable code encoding a predetermined sample characteristic of a MS-integrity-code.

15. Use of a predetermined sample characteristic and a mass spectrometry system configured to provide mass spectrometer data, wherein the use is in cryptography.
